# EUROPEAN PATENT APPLICATION

(11) **EP 1 493 816 A1**
(43) Date of publication of application: **05.01.2005**
(21) Application number: 03077074.7
(22) Date of filing: 02.07.2003
(51) Int. Cl.: C12N 15/63, C12N 15/31, C12N 15/74, C12Q 1/00, C12R 1/23, C12R 1/46, A23C 19/00

(54) **Methods and means for regulating gene expression**

(71) Applicant: Friesland Brands B.V., 7943 PE Meppel (NL)
(72) Inventor: den Hengst, Christiaan Daniel, 9713 VK Groningen (NL); Kuipers, Oscar Paul, 9728 XG Groningen (NL); Kok, Jan, 9714 HL Groningen (NL); Geurts, Johannes Marie Wilhelmus, 7559 NK Hengelo (NL); Nauta, Arjen, 6721 CG Bennekom (NL)
(74) Representative: Prins, Adrianus Willem, Mr. Ir.

(57) **Abstract**

The invention relates to the field of biochemistry, molecular biology and food production. More in particular, the invention relates to methods and means for regulating gene expression. Even more in particular, the invention relates to CodY target sequences.

Herein we show that CodY represses its target genes by binding to specific DNA sequences upstream of the respective genes. A conserved target site was identified by analyzing upstream sequences of derepressed genes in a delta *codYL. lactis* MG1363 derivative, as identified in a DNA micro-array study. The present application furthermore discloses CodY target sequences from other gram-positive bacteria, like *B. subtilis* and *Streptococcus*.

Hence, the invention provides CodY target sequences that may be used in different applications to repress or derepress gene expression.

SEQUENCE LISTING

<110> Friesland Brands B.V.

<120> Methods and means for regulating gene expression

<130> P63590EP00

<140> 03077074.7
<141> 2003-07-02

<160> 192

<170> PatentIn Ver. 2.1

<210> 1
<211> 28
<212> DNA
<213> Artificial Sequence

<220>
<223> Description of Artificial Sequence:
      oligonucleotide opp1

<220>
<221> misc_feature
<222> (1)..(28)

<400> 1
gctctagaca ctcacttgtt ttgcttcc                                    28

<210> 2
<211> 27
<212> DNA
<213> Artificial Sequence

<220>
<223> Description of Artificial Sequence:
      oligonucleotide opp2

<220>
<221> misc_feature
<222> (1)..(27)

<400> 2
aactgcagga aaattcatga acatacc                                     27

<210> 3
<211> 28
<212> DNA
<213> Artificial Sequence

<220>
<223> Description of Artificial Sequence:
      oligonucleotide opp3

<220>
<221> misc_feature
<222> (1)..(28)

<400> 3
aactgcagta aaacaataat aaaagcag                                          28

<210> 4
<211> 27
<212> DNA
<213> Artificial Sequence

<220>
<223> Description of Artificial Sequence:
      oligonucleotide opp4

<220>
<221> misc_feature
<222> (1)..(27)

<400> 4
aactgcagga taataaaatt tggactg                                           27

<210> 5
<211> 27
<212> DNA
<213> Artificial Sequence

<220>
<223> Description of Artificial Sequence:
      oligonucleotide opp14

<220>
<221> misc_feature
<222> (1)..(27)

<400> 5
aactgcagcg taatgttcag aaaattc                                           27

<210> 6
<211> 38
<212> DNA
<213> Artificial Sequence

<220>
<223> Description of Artificial Sequence:
      oligonucleotide opp15 (a)

<220>
<221> misc_feature
<222> (1)..(38)

<400> 6
aactgcagcg taatatttag aaaattcatg aacatacc                               38

<210> 7
<211> 38
<212> DNA
<213> Artificial Sequence

<220>
<223> Description of Artificial Sequence:
      oligonucleotide opp15 (b)

<220>
<221> misc_feature
<222> (1)..(38)

<400> 7
aactgcagcg tactgtgccg aaaattcatg aacatacc                              38

<210> 8
<211> 27
<212> DNA
<213> Artificial Sequence

<220>
<223> Description of Artificial Sequence:
      oligonucleotide cod280A

<220>
<221> misc_feature
<222> (1)..(27)

<400> 8
gggaattcgg attgtctatc tgcctcg                                          27

<210> 9
<211> 36
<212> DNA
<213> Artificial Sequence

<220>
<223> Description of Artificial Sequence:
      oligonucleotide cod280B

<220>
<221> misc_feature
<222> (1)..(36)

<400> 9
gggggatcca gatctgacca tgattacgcc aagctt                                36

<210> 10
<211> 60
<212> DNA
<213> Artificial Sequence

<220>
<223> Description of Artificial Sequence:
      oligonucleotide HC-5

<220>
<221> misc_feature
<222> (1)..(60)

<400> 10
ctagaccacc atggggcatc accatcacca tcacgtggct acattacttg aaaaaacacg 60

<210> 11
<211> 37
<212> DNA
<213> Artificial Sequence

<220>
<223> Description of Artificial Sequence:
      oligonucleotide HC-6

<220>
<221> misc_feature
<222> (1)..(37)

<400> 11
ctagtctaga ttagaaatta cgtccagcaa gtttatc                                37


<210> 12
<211> 8
<212> PRT
<213> Artificial Sequence

<220>
<223> Description of Artificial Sequence: consensus
      sequence wherein X can be any amino acid

<220>
<221> SITE
<222> (1)..(8)

<400> 12
Gly Xaa Xaa Xaa Xaa Gly Xaa Thr
  1               5


<210> 13
<211> 8
<212> PRT
<213> Escherichia coli

<220>
<221> SITE
<222> (1)..(8)
<223> /note="Putative GTP binding motif G1"

<400> 13
Leu Gly Gly Gly Thr Gly Thr Gly
  1               5


<210> 14
<211> 8
<212> PRT
<213> Bacillus subtilis

<220>
<221> SITE
<222> (1)..(8)
<223> /note="Putative GTP binding motif G1"

<400> 14
Gly Gly Glu Arg Leu Gly Thr Leu
1               5


<210> 15
<211> 8
<212> PRT
<213> Bacillus halodurans

<220>
<221> SITE
<222> (1)..(8)
<223> /note="Putative GTP binding motif G1"

<400> 15
Gly Gly Gln Arg Leu Gly Thr Leu
1               5


<210> 16
<211> 8
<212> PRT
<213> Clostridium difficile

<220>
<221> SITE
<222> (1)..(8)
<223> /note="Putative GTP binding motif G1"

<400> 16
Gly Gly Met Arg Leu Gly Ser Leu
1               5


<210> 17
<211> 8
<212> PRT
<213> Clostridium acetobutylicum

<220>
<221> SITE
<222> (1)..(8)
<223> /note="Putative GTP binding motif G1"

<400> 17
Asn Arg Glu Arg Leu Gly Thr Leu
1               5


<210> 18
<211> 8
<212> PRT
<213> Streptococcus pneumoniae

<220>
<221> SITE
<222> (1)..(8)
<223> /note="Putative GTP binding motif G1"

<400> 18
Ser Gly Ile Arg Leu Gly Ser Leu
1               5


<210> 19
<211> 8
<212> PRT
<213> Enterococcus faecalis

<220>
<221> SITE
<222> (1)..(8)
<223> /note="Putative GTP binding motif G1"

<400> 19
Ala Gly Lys Arg Leu Gly Thr Ile
1               5


<210> 20
<211> 8
<212> PRT
<213> Lactococcus lactis

<220>
<221> SITE
<222> (1)..(8)
<223> /note="Putative GTP binding motif G1"

<400> 20
Ser Gly Met Arg Leu Gly Thr Phe
1               5


<210> 21
<211> 4
<212> PRT
<213> Artificial Sequence

<220>
<223> Description of Artificial Sequence: consensus
      sequence wherein X can be any amino acid

<220>
<221> SITE
<222> (1)..(4)

<400> 21
Asp Xaa Xaa Gly
1


<210> 22
<211> 4
<212> PRT
<213> Escherichia coli

<220>
<221> SITE
<222> (1)..(4)

<223> /note="Putative GTP binding motif G3"

<400> 22
Asp Ala Phe Gly
  1


<210> 23
<211> 4
<212> PRT
<213> Bacillus subtilis

<220>
<221> SITE
<222> (1)..(4)
<223> /note="Putative GTP binding motif G3"

<400> 23
Asp Arg Val Gly
  1


<210> 24
<211> 4
<212> PRT
<213> Clostridium difficile

<220>
<221> SITE
<222> (1)..(4)
<223> /note="Putative GTP binding motif G3"

<400> 24
Asp Arg Ile Gly
  1


<210> 25
<211> 4
<212> PRT
<213> Lactococcus lactis

<220>
<221> SITE
<222> (1)..(4)
<223> /note="Putative GTP binding motif G3"

<400> 25
Asp Lys Ile Gly
  1


<210> 26
<211> 4
<212> PRT
<213> Artificial Sequence

<220>
<223> Description of Artificial Sequence: consensus
      sequence wherein X can be any amino acid

<220>
<221> SITE
<222> (1)..(4)
<223> /note="Putative GTP binding motif G4"

<400> 26
Asn Lys Xaa Asp
1

<210> 27
<211> 4
<212> PRT
<213> Escherichia coli

<220>
<221> SITE
<222> (1)..(4)
<223> /note="Putative GTP binding motif G4"

<400> 27
Thr Ser Leu Asp
1

<210> 28
<211> 4
<212> PRT
<213> Bacillus subtilis

<220>
<221> SITE
<222> (1)..(4)
<223> /note="Putative GTP binding motif G4"

<400> 28
Asn Lys Phe Leu
1

<210> 29
<211> 4
<212> PRT
<213> Bacillus stearothermophilus

<220>
<221> SITE
<222> (1)..(4)
<223> /note="Putative GTP binding motif G4"

<400> 29
Asp Lys Phe Leu
1

<210> 30
<211> 4
<212> PRT
<213> Clostridium difficile

<220>

<221> SITE
<222> (1)..(4)
<223> /note="Putative GTP binding motif G4"

<400> 30
Asn Glu Gly Ile
1


<210> 31
<211> 4
<212> PRT
<213> Clostridium acetobutylicum

<220>
<221> SITE
<222> (1)..(4)
<223> /note="Putative GTP binding motif G4"

<400> 31
Ile Leu Asn Asp
1


<210> 32
<211> 4
<212> PRT
<213> Streptococcus pneumoniae

<220>
<221> SITE
<222> (1)..(4)
<223> /note="Putative GTP binding motif G4"

<400> 32
Leu Ile Ser Asp
1


<210> 33
<211> 4
<212> PRT
<213> Enterococcus faecalis

<220>
<221> SITE
<222> (1)..(4)
<223> /note="Putative GTP binding motif G4"

<400> 33
Asn Gln Gln Phe
1


<210> 34
<211> 4
<212> PRT
<213> Staphylococcus aureus

<220>
<221> SITE

<222> (1)..(4)
<223> /note="Putative GTP binding motif G4"

<400> 34
Glu Lys Gly Ile
1


<210> 35
<211> 4
<212> PRT
<213> Lactococcus lactis

<220>
<221> SITE
<222> (1)..(4)
<223> /note="Putative GTP binding motif G4"

<400> 35
Thr Gly Leu Phe
1


<210> 36
<211> 26
<212> DNA
<213> Artificial Sequence

<220>
<223> Description of Artificial Sequence: presence of
      the putative CodY box in yreE

<220>
<221> misc_feature
<222> (1)..(26)

<400> 36
taattttctg ataatatagt caattt                                          26


<210> 37
<211> 26
<212> DNA
<213> Artificial Sequence

<220>
<223> Description of Artificial Sequence: presence of
      the putative CodY box in ctrA

<220>
<221> misc_feature
<222> (1)..(26)

<400> 37
taatttactg acaagtctgt cagtaa                                          26


<210> 38
<211> 26
<212> DNA
<213> Artificial Sequence

<220>
<223> Description of Artificial Sequence: presence of
      the putative CodY box in yciC

<220>
<221> misc_feature
<222> (1)..(26)

<400> 38
taatttactg acaaaattat cagaac                                          26


<210> 39
<211> 26
<212> DNA
<213> Artificial Sequence

<220>
<223> Description of Artificial Sequence: presence of
      the putative CodY box in optA

<220>
<221> misc_feature
<222> (1)..(26)

<400> 39
aaattttctg acaataataa aaattg                                          26


<210> 40
<211> 26
<212> DNA
<213> Artificial Sequence

<220>
<223> Description of Artificial Sequence: presence of
      the putative CodY box in optS

<220>
<221> misc_feature
<222> (1)..(26)

<400> 40
aaattatcag aaaaatacaa caatat                                          26


<210> 41
<211> 26
<212> DNA
<213> Artificial Sequence

<220>
<223> Description of Artificial Sequence: presence of
      the putative CodY box in optS

<220>
<221> misc_feature
<222> (1)..(26)

<400> 41

taattttcag aataatatga aaattc                                    26

<210> 42
<211> 26
<212> DNA
<213> Artificial Sequence

<220>
<223> Description of Artificial Sequence: presence of
      the putative CodY box in parA

<220>
<221> misc_feature
<222> (1)..(26)

<400> 42
taatttactg atagatttgt cagtaa                                    26

<210> 43
<211> 26
<212> DNA
<213> Artificial Sequence

<220>
<223> Description of Artificial Sequence: presence of
      the putative CodY box in aroF

<220>
<221> misc_feature
<222> (1)..(26)

<400> 43
taatttactg acagttctgt cagtaa                                    26

<210> 44
<211> 26
<212> DNA
<213> Artificial Sequence

<220>
<223> Description of Artificial Sequence: presence of
      the putative CodY box in vacB1

<220>
<221> misc_feature
<222> (1)..(26)

<400> 44
aaatttactg acaaaaaaga taatgg                                    26

<210> 45
<211> 26
<212> DNA
<213> Artificial Sequence

<220>
<223> Description of Artificial Sequence: presence of

the putative CodY box in optA

<220>
<221> misc_feature
<222> (1)..(26)

<400> 45
taattttcag aaaacataac cattat                                          26


<210> 46
<211> 26
<212> DNA
<213> Artificial Sequence

<220>
<223> Description of Artificial Sequence: presence of
      the putative CodY box in ypaG

<220>
<221> misc_feature
<222> (1)..(26)

<400> 46
gaattttatg aaaaaaatat taattg                                          26


<210> 47
<211> 26
<212> DNA
<213> Artificial Sequence

<220>
<223> Description of Artificial Sequence: presence of
      the putative CodY box in yiaB

<220>
<221> misc_feature
<222> (1)..(26)

<400> 47
gaatttactg acgaatctat cattaa                                          26


<210> 48
<211> 26
<212> DNA
<213> Artificial Sequence

<220>
<223> Description of Artificial Sequence: presence of
      the putative CodY box in ysdC

<220>
<221> misc_feature
<222> (1)..(26)

<400> 48
tcattctctg acaaatctgt cagtaa                                          26

<210> 49
<211> 26
<212> DNA
<213> Artificial Sequence

<220>
<223> Description of Artificial Sequence: presence of
      the putative CodY box in hemK

<220>
<221> misc_feature
<222> (1)..(26)

<400> 49
aaatttactg acaagcttgt tagtat                                          26


<210> 50
<211> 26
<212> DNA
<213> Artificial Sequence

<220>
<223> Description of Artificial Sequence: presence of
      the putative CodY box in prfC

<220>
<221> misc_feature
<222> (1)..(26)

<400> 50
aaatttaatg ataaaacaat tagttt                                          26


<210> 51
<211> 26
<212> DNA
<213> Artificial Sequence

<220>
<223> Description of Artificial Sequence: presence of
      the putative CodY box in yugB

<220>
<221> misc_feature
<222> (1)..(26)

<400> 51
aaagttactg acaaatctgt cagtaa                                          26


<210> 52
<211> 26
<212> DNA
<213> Artificial Sequence

<220>
<223> Description of Artificial Sequence: presence of
      the putative CodY box in murD

<220>

<221> misc_feature
<222> (1)..(26)

<400> 52
ttatttactg acaagtctgt cagtaa                                            26


<210> 53
<211> 26
<212> DNA
<213> Artificial Sequence

<220>
<223> Description of Artificial Sequence: presence of
      the putative CodY box in ywdG

<220>
<221> misc_feature
<222> (1)..(26)

<400> 53
tattttactg acaaaaaaat aagttt                                            26


<210> 54
<211> 26
<212> DNA
<213> Artificial Sequence

<220>
<223> Description of Artificial Sequence: presence of
      the putative CodY box in parC

<220>
<221> misc_feature
<222> (1)..(26)

<400> 54
taatttactg acagctttgt cagtaa                                            26


<210> 55
<211> 26
<212> DNA
<213> Artificial Sequence

<220>
<223> Description of Artificial Sequence: presence of
      the putative CodY box in pepC

<220>
<221> misc_feature
<222> (1)..(26)

<400> 55
aaatttactg acagagctgt cagtaa                                            26


<210> 56
<211> 26
<212> DNA

<213> Artificial Sequence

<220>
<223> Description of Artificial Sequence: presence of
      the putative CodY box in mutM

<220>
<221> misc_feature
<222> (1)..(26)

<400> 56
aaatttactg acagacttgt tagtaa                                            26


<210> 57
<211> 26
<212> DNA
<213> Artificial Sequence

<220>
<223> Description of Artificial Sequence: presence of
      the putative CodY box in rgpAB

<220>
<221> misc_feature
<222> (1)..(26)

<400> 57
aaatttactg acaactttgt cagaag                                            26


<210> 58
<211> 26
<212> DNA
<213> Artificial Sequence

<220>
<223> Description of Artificial Sequence: presence of
      the putative CodY box in vacB1

<220>
<221> misc_feature
<222> (1)..(26)

<400> 58
aaaatgtctg ataaaatgat taatac                                            26


<210> 59
<211> 26
<212> DNA
<213> Artificial Sequence

<220>
<223> Description of Artificial Sequence: presence of
      the putative CodY box in recN

<220>
<221> misc_feature
<222> (1)..(26)

<400> 59
taatttactg acagaatttt aaattt                                          26


<210> 60
<211> 26
<212> DNA
<213> Artificial Sequence

<220>
<223> Description of Artificial Sequence: presence of
      the putative CodY box in llrH

<220>
<221> misc_feature
<222> (1)..(26)

<400> 60
aaaattacta acaaaactgt tagtaa                                          26


<210> 61
<211> 15
<212> DNA
<213> Artificial Sequence

<220>
<223> Description of Artificial Sequence: presence of
      putative CodY box in yufN

<220>
<221> misc_feature
<222> (1)..(15)

<400> 61
attatcagaa aattt                                                      15


<210> 62
<211> 15
<212> DNA
<213> Artificial Sequence

<220>
<223> Description of Artificial Sequence: presence of
      putative CodY box in citB

<220>
<221> misc_feature
<222> (1)..(15)

<400> 62
attgtgagaa aattg                                                      15


<210> 63
<211> 15
<212> DNA
<213> Artificial Sequence

<220>

<223> Description of Artificial Sequence: presence of
      putative CodY box in dppA

<220>
<221> misc_feature
<222> (1)..(15)

<400> 63
tttgttagaa tattc                                                    15


<210> 64
<211> 15
<212> DNA
<213> Artificial Sequence

<220>
<223> Description of Artificial Sequence: presence of
      putative CodY box in hutP

<220>
<221> misc_feature
<222> (1)..(15)

<400> 64
gttatcagaa ttttt                                                    15


<210> 65
<211> 15
<212> DNA
<213> Artificial Sequence

<220>
<223> Description of Artificial Sequence: presence of
      putative CodY box in yxbC

<220>
<221> misc_feature
<222> (1)..(15)

<400> 65
attatcagag gatta                                                    15


<210> 66
<211> 15
<212> DNA
<213> Artificial Sequence

<220>
<223> Description of Artificial Sequence: presence of
      putative CodY box in yurPQ

<220>
<221> misc_feature
<222> (1)..(15)

<400> 66
aatttcagaa aataa                                                    15

<210> 67
<211> 15
<212> DNA
<213> Artificial Sequence

<220>
<223> Description of Artificial Sequence: presence of
      putative CodY box in ycgM

<220>
<221> misc_feature
<222> (1)..(15)

<400> 67
attttgagga tattg                                                        15


<210> 68
<211> 15
<212> DNA
<213> Artificial Sequence

<220>
<223> Description of Artificial Sequence: presence of
      putative CodY box in yhjC

<220>
<221> misc_feature
<222> (1)..(15)

<400> 68
aatttcagac aattc                                                        15


<210> 69
<211> 15
<212> DNA
<213> Artificial Sequence

<220>
<223> Description of Artificial Sequence: presence of
      putative CodY box in ybgE

<220>
<221> misc_feature
<222> (1)..(15)

<400> 69
atattctgaa attta                                                        15


<210> 70
<211> 15
<212> DNA
<213> Artificial Sequence

<220>
<223> Description of Artificial Sequence: presence of
      putative CodY box in ykbA

<220>
<221> misc_feature
<222> (1)..(15)

<400> 70
tttatcaaaa aagtc                                                        15


<210> 71
<211> 15
<212> DNA
<213> Artificial Sequence

<220>
<223> Description of Artificial Sequence: presence of
      putative CodY box in ggaA

<220>
<221> misc_feature
<222> (1)..(15)

<400> 71
attttcagca aaaaa                                                        15


<210> 72
<211> 15
<212> DNA
<213> Artificial Sequence

<220>
<223> Description of Artificial Sequence: presence of
      putative CodY box in ycgM

<220>
<221> misc_feature
<222> (1)..(15)

<400> 72
ataatcagaa tcttt                                                        15


<210> 73
<211> 15
<212> DNA
<213> Artificial Sequence

<220>
<223> Description of Artificial Sequence: presence of
      putative CodY box in yoaD

<220>
<221> misc_feature
<222> (1)..(15)

<400> 73
tttttatgaa aaata                                                        15


<210> 74
<211> 15

<212> DNA
<213> Artificial Sequence

<220>
<223> Description of Artificial Sequence: presence of
      putative CodY box in guaB

<220>
<221> misc_feature
<222> (1)..(15)

<400> 74
gttatctaaa tattt                                                        15


<210> 75
<211> 15
<212> DNA
<213> Artificial Sequence

<220>
<223> Description of Artificial Sequence: presence of
      putative CodY box in ilvD

<220>
<221> misc_feature
<222> (1)..(15)

<400> 75
attgtcaaaa taaaa                                                        15


<210> 76
<211> 15
<212> DNA
<213> Artificial Sequence

<220>
<223> Description of Artificial Sequence: presence of
      putative CodY box in yxbBC

<220>
<221> misc_feature
<222> (1)..(15)

<400> 76
attgacagaa ttatc                                                        15


<210> 77
<211> 15
<212> DNA
<213> Artificial Sequence

<220>
<223> Description of Artificial Sequence: presence of
      putative CodY box in rocA

<220>
<221> misc_feature
<222> (1)..(15)

<400> 77
tttttcagca aaaga                                                        15


<210> 78
<211> 15
<212> DNA
<213> Artificial Sequence

<220>
<223> Description of Artificial Sequence: presence of
      putative CodY box in yhdG

<220>
<221> misc_feature
<222> (1)..(15)

<400> 78
tttttctaac aattt                                                        15


<210> 79
<211> 15
<212> DNA
<213> Artificial Sequence

<220>
<223> Description of Artificial Sequence: presence of
      putative CodY box in yusC

<220>
<221> misc_feature
<222> (1)..(15)

<400> 79
ttttgcagaa aaaac                                                        15


<210> 80
<211> 15
<212> DNA
<213> Artificial Sequence

<220>
<223> Description of Artificial Sequence: presence of
      putative CodY box in yusC

<220>
<221> misc_feature
<222> (1)..(15)

<400> 80
tttcttagaa tagtg                                                        15


<210> 81
<211> 15
<212> DNA
<213> Artificial Sequence

<220>
<223> Description of Artificial Sequence: presence of
      the putative CodY box in ydjJ

<220>
<221> misc_feature
<222> (1)..(15)

<400> 81
attttcagaa attta                                                        15


<210> 82
<211> 15
<212> DNA
<213> Artificial Sequence

<220>
<223> Description of Artificial Sequence: presence of
      the putative CodY box in ytkC

<220>
<221> misc_feature
<222> (1)..(15)

<400> 82
tttttcagaa aaatg                                                        15


<210> 83
<211> 15
<212> DNA
<213> Artificial Sequence

<220>
<223> Description of Artificial Sequence: presence of
      the putative CodY box in lytE

<220>
<221> misc_feature
<222> (1)..(15)

<400> 83
tttttcagaa aaatc                                                        15


<210> 84
<211> 15
<212> DNA
<213> Artificial Sequence

<220>
<223> Description of Artificial Sequence: presence of
      the putative CodY box in yoaC

<220>
<221> misc_feature
<222> (1)..(15)

<400> 84
attgtctgaa tatta                                                        15

<210> 85
<211> 15
<212> DNA
<213> Artificial Sequence

<220>
<223> Description of Artificial Sequence: presence of
      the putative CodY box in ytnA

<220>
<221> misc_feature
<222> (1)..(15)

<400> 85
tttttctgaa tattc                                                    15


<210> 86
<211> 15
<212> DNA
<213> Artificial Sequence

<220>
<223> Description of Artificial Sequence: presence of
      the putative CodY box in aldY

<220>
<221> misc_feature
<222> (1)..(15)

<400> 86
attttcggaa aattt                                                    15


<210> 87
<211> 15
<212> DNA
<213> Artificial Sequence

<220>
<223> Description of Artificial Sequence: presence of
      the putative CodY box in glnQ

<220>
<221> misc_feature
<222> (1)..(15)

<400> 87
attttcagaa aagtt                                                    15


<210> 88
<211> 15
<212> DNA
<213> Artificial Sequence

<220>
<223> Description of Artificial Sequence: presence of
      the putative CodY box in ykuW

<220>
<221> misc_feature
<222> (1)..(15)

<400> 88
attttcagaa aataa                                                        15


<210> 89
<211> 15
<212> DNA
<213> Artificial Sequence

<220>
<223> Description of Artificial Sequence: presence of
      the putative CodY box in yndN

<220>
<221> misc_feature
<222> (1)..(15)

<400> 89
attttcagta tattt                                                        15


<210> 90
<211> 15
<212> DNA
<213> Artificial Sequence

<220>
<223> Description of Artificial Sequence: presence of
      the putative CodY box in yheI

<220>
<221> misc_feature
<222> (1)..(15)

<400> 90
tttatcagaa aaata                                                        15


<210> 91
<211> 15
<212> DNA
<213> Artificial Sequence

<220>
<223> Description of Artificial Sequence: presence of
      the putative CodY box in ykuM

<220>
<221> misc_feature
<222> (1)..(15)

<400> 91
attttcagga aattc                                                        15


<210> 92

<211> 15
<212> DNA
<213> Artificial Sequence

<220>
<223> Description of Artificial Sequence: presence of
      the putative CodY box in gbsA

<220>
<221> misc_feature
<222> (1)..(15)

<400> 92
attttcagaa caatt                                                        15


<210> 93
<211> 15
<212> DNA
<213> Artificial Sequence

<220>
<223> Description of Artificial Sequence: presence of
      the putative CodY box in ynaC

<220>
<221> misc_feature
<222> (1)..(15)

<400> 93
attttcagaa aatca                                                        15


<210> 94
<211> 15
<212> DNA
<213> Artificial Sequence

<220>
<223> Description of Artificial Sequence: presence of
      the putative CodY box in yqiQ

<220>
<221> misc_feature
<222> (1)..(15)

<400> 94
attgtcagaa aactt                                                        15


<210> 95
<211> 15
<212> DNA
<213> Artificial Sequence

<220>
<223> Description of Artificial Sequence: presence of
      the putative CodY box in ggaA

<220>
<221> misc_feature

<222> (1)..(15)

<400> 95
attttcagaa ttata                                                        15


<210> 96
<211> 15
<212> DNA
<213> Artificial Sequence

<220>
<223> Description of Artificial Sequence: presence of
      the putative CodY box in yurY

<220>
<221> misc_feature
<222> (1)..(15)

<400> 96
tttttcggaa tattc                                                        15


<210> 97
<211> 15
<212> DNA
<213> Artificial Sequence

<220>
<223> Description of Artificial Sequence: presence of
      the putative CodY box in ureA

<220>
<221> misc_feature
<222> (1)..(15)

<400> 97
tttatctgaa aattt                                                        15


<210> 98
<211> 15
<212> DNA
<213> Artificial Sequence

<220>
<223> Description of Artificial Sequence: presence of
      the putative CodY box in leuS

<220>
<221> misc_feature
<222> (1)..(15)

<400> 98
atattcagaa tattc                                                        15


<210> 99
<211> 15
<212> DNA
<213> Artificial Sequence

<220>
<223> Description of Artificial Sequence: presence of
      the putative CodY box in yurO

<220>
<221> misc_feature
<222> (1)..(15)

<400> 99
attttctgaa atttc                                                        15


<210> 100
<211> 15
<212> DNA
<213> Artificial Sequence

<220>
<223> Description of Artificial Sequence: presence of
      the putative CodY box in ykrQ

<220>
<221> misc_feature
<222> (1)..(15)

<400> 100
attttctgaa attta                                                        15


<210> 101
<211> 15
<212> DNA
<213> Artificial Sequence

<220>
<223> Description of Artificial Sequence: presence of
      the putative CodY box in yvcC

<220>
<221> misc_feature
<222> (1)..(15)

<400> 101
tttttcagta tattt                                                        15


<210> 102
<211> 15
<212> DNA
<213> Artificial Sequence

<220>
<223> Description of Artificial Sequence: presence of
      the putative CodY box in thiC

<220>
<221> misc_feature
<222> (1)..(15)

<400> 102

tttttcagca aattt                                                    15


<210> 103
<211> 15
<212> DNA
<213> Artificial Sequence

<220>
<223> Description of Artificial Sequence: presence of
      the putative CodY box in yvaV

<220>
<221> misc_feature
<222> (1)..(15)

<400> 103
tttttcagac aattg                                                    15


<210> 104
<211> 15
<212> DNA
<213> Artificial Sequence

<220>
<223> Description of Artificial Sequence: presence of
      the putative CodY box in fliR

<220>
<221> misc_feature
<222> (1)..(15)

<400> 104
attgtcagca tattt                                                    15


<210> 105
<211> 15
<212> DNA
<213> Artificial Sequence

<220>
<223> Description of Artificial Sequence: presence of
      the putative CodY box in yokA

<220>
<221> misc_feature
<222> (1)..(15)

<400> 105
tttttcataa aattt                                                    15


<210> 106
<211> 15
<212> DNA
<213> Artificial Sequence

<220>
<223> Description of Artificial Sequence: presence of

the putative CodY box in yqeY

<220>
<221> misc_feature
<222> (1)..(15)

<400> 106
tttttcataa aattc                                                        15


<210> 107
<211> 15
<212> DNA
<213> Artificial Sequence

<220>
<223> Description of Artificial Sequence: presence of
      the putative CodY box in yjcL

<220>
<221> misc_feature
<222> (1)..(15)

<400> 107
attgttagaa aatta                                                        15


<210> 108
<211> 15
<212> DNA
<213> Artificial Sequence

<220>
<223> Description of Artificial Sequence: presence of
      the putative CodY box in rapE

<220>
<221> misc_feature
<222> (1)..(15)

<400> 108
actttcagaa tattt                                                        15


<210> 109
<211> 15
<212> DNA
<213> Artificial Sequence

<220>
<223> Description of Artificial Sequence: presence of
      the putative CodY box in bfmBB

<220>
<221> misc_feature
<222> (1)..(15)

<400> 109
tttttctgaa taatt                                                        15

<210> 110
<211> 26
<212> DNA
<213> Artificial Sequence

<220>
<223> Description of Artificial Sequence: presence of
      the putative CodY box in ppmA

<220>
<221> misc_feature
<222> (1)..(26)

<400> 110
tgattttcag aaaaatttaa gaaaaa                                            26


<210> 111
<211> 26
<212> DNA
<213> Artificial Sequence

<220>
<223> Description of Artificial Sequence: presence of
      the putative CodY box in Leu-A

<220>
<221> misc_feature
<222> (1)..(26)

<400> 111
atattttctg aaaatttctt cagtaa                                            26


<210> 112
<211> 26
<212> DNA
<213> Artificial Sequence

<220>
<223> Description of Artificial Sequence: presence of
      the putative CodY box in hsds

<220>
<221> misc_feature
<222> (1)..(26)

<400> 112
ttattatcag ataattttat caatcg                                            26


<210> 113
<211> 26
<212> DNA
<213> Artificial Sequence

<220>
<223> Description of Artificial Sequence: presence of
      the putative CodY box in livJ

<220>

<221> misc_feature
<222> (1)..(26)

<400> 113
caattttctg ataattcggt atattc                                          26


<210> 114
<211> 26
<212> DNA
<213> Artificial Sequence

<220>
<223> Description of Artificial Sequence: presence of
      the putative CodY box in ilvE

<220>
<221> misc_feature
<222> (1)..(26)

<400> 114
gaattttctg aaaattacaa aatata                                          26


<210> 115
<211> 26
<212> DNA
<213> Artificial Sequence

<220>
<223> Description of Artificial Sequence: presence of
      the putative CodY box in gapN

<220>
<221> misc_feature
<222> (1)..(26)

<400> 115
ttattttctg aaaatttggt aaaata                                          26


<210> 116
<211> 26
<212> DNA
<213> Artificial Sequence

<220>
<223> Description of Artificial Sequence: presence of
      the putative CodY box in mefE

<220>
<221> misc_feature
<222> (1)..(26)

<400> 116
   tcattttttg aaaaaatgat tattac                                           26


<210> 117
<211> 26
<212> DNA

<213> Artificial Sequence

<220>
<223> Description of Artificial Sequence: presence of
      the putative CodY box in IS1381

<220>
<221> misc_feature
<222> (1)..(26)

<400> 117
cattttcag aaaattcttt tatttc                                          26


<210> 118
<211> 26
<212> DNA
<213> Artificial Sequence

<220>
<223> Description of Artificial Sequence: presence of
      the putative CodY box in ugd

<220>
<221> misc_feature
<222> (1)..(26)

<400> 118
acattttctg aaattaaaaa taatat                                         26


<210> 119
<211> 26
<212> DNA
<213> Artificial Sequence

<220>
<223> Description of Artificial Sequence: presence of
      the putative CodY box in hemK

<220>
<221> misc_feature
<222> (1)..(26)

<400> 119
gaaatttctg aaaaatatga tataat                                         26


<210> 120
<211> 26
<212> DNA
<213> Artificial Sequence

<220>
<223> Description of Artificial Sequence: presence of
      the putative CodY box in spr1649

<220>
<221> misc_feature
<222> (1)..(26)

<400> 120
aaattatttg ataattctat aatttc                                          26


<210> 121
<211> 26
<212> DNA
<213> Artificial Sequence

<220>
<223> Description of Artificial Sequence: presence of
      the putative CodY box in spr1765

<220>
<221> misc_feature
<222> (1)..(26)

<400> 121
aatttatctg aaaaaacgaa aaatat                                          26


<210> 122
<211> 26
<212> DNA
<213> Artificial Sequence

<220>
<223> Description of Artificial Sequence: presence of
      the putative CodY box in glgB

<220>
<221> misc_feature
<222> (1)..(26)

<400> 122
caatttttg aaaaaatatt gattta                                           26


<210> 123
<211> 26
<212> DNA
<213> Artificial Sequence

<220>
<223> Description of Artificial Sequence: presence of
      the putative CodY box in spr2010

<220>
<221> misc_feature
<222> (1)..(26)

<400> 123
tatttttctg aaaactctga tataaa                                          26


<210> 124
<211> 26
<212> DNA
<213> Artificial Sequence

<220>

<223> Description of Artificial Sequence: presence of
      the putative CodY box in spr0128

<220>
<221> misc_feature
<222> (1)..(26)

<400> 124
tcattttcag ataaggataa aaattg                                            26


<210> 125
<211> 26
<212> DNA
<213> Artificial Sequence

<220>
<223> Description of Artificial Sequence: presence of
      the putative CodY box in ABC-NDB

<220>
<221> misc_feature
<222> (1)..(26)

<400> 125
gaattgacag atgaatttgt taagaa                                            26


<210> 126
<211> 26
<212> DNA
<213> Artificial Sequence

<220>
<223> Description of Artificial Sequence: presence of
      the putative CodY box in spr1149

<220>
<221> misc_feature
<222> (1)..(26)

<400> 126
aaattgaatg aaaagtataa aattaa                                            26


<210> 127
<211> 26
<212> DNA
<213> Artificial Sequence

<220>
<223> Description of Artificial Sequence: presence of
      the putative CodY box in rgg/spr1934

<220>
<221> misc_feature
<222> (1)..(26)

<400> 127
aaattgtcag aattatgaga aaatag                                            26

<210> 128
<211> 26
<212> DNA
<213> Artificial Sequence

<220>
<223> Description of Artificial Sequence: presence of
      the putative CodY box in gidA

<220>
<221> misc_feature
<222> (1)..(26)

<400> 128
cattttactg aagaatacga tattat                                          26


<210> 129
<211> 26
<212> DNA
<213> Artificial Sequence

<220>
<223> Description of Artificial Sequence: presence of
      the putative CodY box in abc-n/p

<220>
<221> misc_feature
<222> (1)..(26)

<400> 129
aaatttctag ataagttaat aattaa                                          26


<210> 130
<211> 26
<212> DNA
<213> Artificial Sequence

<220>
<223> Description of Artificial Sequence: presence of
      the putative CodY box in spr0119

<220>
<221> misc_feature
<222> (1)..(26)

<400> 130
atattctgtg aaaaataaaa tagtat                                          26


<210> 131
<211> 26
<212> DNA
<213> Artificial Sequence

<220>
<223> Description of Artificial Sequence: presence of
      the putative CodY box in hsdS 2X

<220>
<221> misc_feature
<222> (1)..(26)

<400> 131
gaatttccag ataaactaaa aaaatc                                          26


<210> 132
<211> 26
<212> DNA
<213> Artificial Sequence

<220>
<223> Description of Artificial Sequence: presence of
      the putative CodY box in spr0607

<220>
<221> misc_feature
<222> (1)..(26)

<400> 132
acattatctg aaaaattaaa ctataa                                          26


<210> 133
<211> 26
<212> DNA
<213> Artificial Sequence

<220>
<223> Description of Artificial Sequence: presence of
      the putative CodY box in rpmI

<220>
<221> misc_feature
<222> (1)..(26)

<400> 133
taattgtcag aaagttaaat aaagga                                          26


<210> 134
<211> 26
<212> DNA
<213> Artificial Sequence

<220>
<223> Description of Artificial Sequence: presence of
      the putative CodY box in spnIM

<220>
<221> misc_feature
<222> (1)..(26)

<400> 134
aaattatcta ataacaaaaa tattat                                          26


<210> 135
<211> 26

<212> DNA
<213> Artificial Sequence

<220>
<223> Description of Artificial Sequence: presence of
      the putative CodY box in spnIM

<220>
<221> misc_feature
<222> (1)..(26)

<400> 135
ttatttacag aaagaacaaa aaatgc                                          26


<210> 136
<211> 26
<212> DNA
<213> Artificial Sequence

<220>
<223> Description of Artificial Sequence: presence of
      the putative CodY box in ilvD

<220>
<221> misc_feature
<222> (1)..(26)

<400> 136
gaattttcag aaaattctat acgcat                                          26


<210> 137
<211> 26
<212> DNA
<213> Artificial Sequence

<220>
<223> Description of Artificial Sequence: presence of
      the putative CodY box in arcA

<220>
<221> misc_feature
<222> (1)..(26)

<400> 137
atatttttg aaaaattttt taaaaa                                           26


<210> 138
<211> 26
<212> DNA
<213> Artificial Sequence

<220>
<223> Description of Artificial Sequence: presence of
      the putative CodY box in ABC-NBD

<220>
<221> misc_feature
<222> (1)..(26)

<400> 138
aaattgacaa ataaaaattt gaatat                                          26


<210> 139
<211> 26
<212> DNA
<213> Artificial Sequence

<220>
<223> Description of Artificial Sequence: presence of
      the putative CodY box in mfd

<220>
<221> misc_feature
<222> (1)..(26)

<400> 139
tactttacag aagaattaca gaatag                                          26


<210> 140
<211> 26
<212> DNA
<213> Artificial Sequence

<220>
<223> Description of Artificial Sequence: presence of
      the putative CodY box in spr0157

<220>
<221> misc_feature
<222> (1)..(26)

<400> 140
gaattttcag aataatctgt atatgt                                          26


<210> 141
<211> 26
<212> DNA
<213> Artificial Sequence

<220>
<223> Description of Artificial Sequence: presence of
      the putative CodY box in murM

<220>
<221> misc_feature
<222> (1)..(26)

<400> 141
gaatttcctg aaaatctggc tattat                                          26


<210> 142
<211> 26
<212> DNA
<213> Artificial Sequence

<220>
<223> Description of Artificial Sequence: presence of
      the putative CodY box in zwf

<220>
<221> misc_feature
<222> (1)..(26)

<400> 142
gatttttcgg aaaattatgt tagaat                                          26


<210> 143
<211> 26
<212> DNA
<213> Artificial Sequence

<220>
<223> Description of Artificial Sequence: presence of
      the putative CodY box in licD1

<220>
<221> misc_feature
<222> (1)..(26)

<400> 143
caatatttag aaaaaaaaga aattaa                                          26


<210> 144
<211> 26
<212> DNA
<213> Artificial Sequence

<220>
<223> Description of Artificial Sequence: presence of
      the putative CodY box in spr1403

<220>
<221> misc_feature
<222> (1)..(26)

<400> 144
atattttctg aacaattaat atattc                                          26


<210> 145
<211> 26
<212> DNA
<213> Artificial Sequence

<220>
<223> Description of Artificial Sequence: presence of
      the putative CodY box in relA

<220>
<221> misc_feature
<222> (1)..(26)

<400> 145
cattttgcgg aaaattgagt aaatat                                          26

<210> 146
<211> 26
<212> DNA
<213> Artificial Sequence

<220>
<223> Description of Artificial Sequence: presence of
      the putative CodY box in spr1650

<220>
<221> misc_feature
<222> (1)..(26)

<400> 146
taattttctg atttttgta aaataa                                          26

<210> 147
<211> 26
<212> DNA
<213> Artificial Sequence

<220>
<223> Description of Artificial Sequence: presence of
      the putative CodY box in pcpA

<220>
<221> misc_feature
<222> (1)..(26)

<400> 147
ttattttcta atagatataa aattat                                         26

<210> 148
<211> 26
<212> DNA
<213> Artificial Sequence

<220>
<223> Description of Artificial Sequence: presence of
      the putative CodY box in rsuA

<220>
<221> misc_feature
<222> (1)..(26)

<400> 148
taaatttcag attgatgaaa aaatag                                         26

<210> 149
<211> 26
<212> DNA
<213> Artificial Sequence

<220>
<223> Description of Artificial Sequence: presence of
      the putative CodY box in ptsG

<220>
<221> misc_feature
<222> (1)..(26)

<400> 149
aattttttag aaaaaagtgt aatttt                                          26


<210> 150
<211> 26
<212> DNA
<213> Artificial Sequence

<220>
<223> Description of Artificial Sequence: presence of
      the putative CodY box in hsdS

<220>
<221> misc_feature
<222> (1)..(26)

<400> 150
aaattcactg aaagtttaaa tatgac                                          26


<210> 151
<211> 26
<212> DNA
<213> Artificial Sequence

<220>
<223> Description of Artificial Sequence: presence of
      the putative CodY box in spr0803

<220>
<221> misc_feature
<222> (1)..(26)

<400> 151
gaattgccag actattttaa tactat                                          26


<210> 152
<211> 26
<212> DNA
<213> Artificial Sequence

<220>
<223> Description of Artificial Sequence: presence of
      the putative CodY box in spr0826

<220>
<221> misc_feature
<222> (1)..(26)

<400> 152
agattttaag taaaatttat tagtaa                                          26


<210> 153

<211> 26
<212> DNA
<213> Artificial Sequence

<220>
<223> Description of Artificial Sequence: presence of
      the putative CodY box in flaV

<220>
<221> misc_feature
<222> (1)..(26)

<400> 153
aaattttaga aaaaattaaa gaatac                                          26


<210> 154
<211> 26
<212> DNA
<213> Artificial Sequence

<220>
<223> Description of Artificial Sequence: presence of
      the putative CodY box in ABC-NP

<220>
<221> misc_feature
<222> (1)..(26)

<400> 154
caattatctg atcatctgaa aaatat                                          26


<210> 155
<211> 15
<212> DNA
<213> Artificial Sequence

<220>
<223> Description of Artificial Sequence: presence of
      the putative CodY box in gbs1632

<220>
<221> misc_feature
<222> (1)..(15)

<400> 155
attatcagaa tattg                                                      15


<210> 156
<211> 15
<212> DNA
<213> Artificial Sequence

<220>
<223> Description of Artificial Sequence: presence of
      the putative CodY box in gbs1105

<220>
<221> misc_feature

<222> (1)..(15)

<400> 156
attttcagaa aaata                                                        15


<210> 157
<211> 15
<212> DNA
<213> Artificial Sequence

<220>
<223> Description of Artificial Sequence: presence of
      the putative CodY box in gbs0662

<220>
<221> misc_feature
<222> (1)..(15)

<400> 157
tttatcagaa aattt                                                        15


<210> 158
<211> 15
<212> DNA
<213> Artificial Sequence

<220>
<223> Description of Artificial Sequence: presence of
      the putative CodY box in gbs2002

<220>
<221> misc_feature
<222> (1)..(15)

<400> 158
attttctgaa tattc                                                        15


<210> 159
<211> 15
<212> DNA
<213> Artificial Sequence

<220>
<223> Description of Artificial Sequence: presence of
      the putative CodY box in gbs1489

<220>
<221> misc_feature
<222> (1)..(15)

<400> 159
tttttctgaa tattt                                                        15


<210> 160
<211> 15
<212> DNA
<213> Artificial Sequence

<220>
<223> Description of Artificial Sequence: presence of
      the putative CodY box in gbs0054

<220>
<221> misc_feature
<222> (1)..(15)

<400> 160
attttcaaaa aattt                                                        15


<210> 161
<211> 15
<212> DNA
<213> Artificial Sequence

<220>
<223> Description of Artificial Sequence: presence of
      the putative CodY box in gbs0008

<220>
<221> misc_feature
<222> (1)..(15)

<400> 161
attttcaaaa aattg                                                        15


<210> 162
<211> 15
<212> DNA
<213> Artificial Sequence

<220>
<223> Description of Artificial Sequence: presence of
      the putative CodY box in gbs1406

<220>
<221> misc_feature
<222> (1)..(15)

<400> 162
attgtcagaa ttttc                                                        15


<210> 163
<211> 15
<212> DNA
<213> Artificial Sequence

<220>
<223> Description of Artificial Sequence: presence of
      the putative CodY box in gbs0144

<220>
<221> misc_feature
<222> (1)..(15)

<400> 163

attatctgaa aattt                                                    15

<210> 164
<211> 15
<212> DNA
<213> Artificial Sequence

<220>
<223> Description of Artificial Sequence: presence of
      the putative CodY box in gbs0898

<220>
<221> misc_feature
<222> (1)..(15)

<400> 164
attatctgaa tatta                                                    15

<210> 165
<211> 15
<212> DNA
<213> Artificial Sequence

<220>
<223> Description of Artificial Sequence: presence of
      the putative CodY box in gbs2007

<220>
<221> misc_feature
<222> (1)..(15)

<400> 165
attttcagta tattc                                                    15

<210> 166
<211> 15
<212> DNA
<213> Artificial Sequence

<220>
<223> Description of Artificial Sequence: presence of
      the putative CodY box in gbs0235

<220>
<221> misc_feature
<222> (1)..(15)

<400> 166
attttcagaa aatgt                                                    15

<210> 167
<211> 15
<212> DNA
<213> Artificial Sequence

<220>
<223> Description of Artificial Sequence: presence of

the putative CodY box in gbs0577

<220>
<221> misc_feature
<222> (1)..(15)

<400> 167
attatcagaa gattt                                                        15


<210> 168
<211> 15
<212> DNA
<213> Artificial Sequence

<220>
<223> Description of Artificial Sequence: presence of
      the putative CodY box in gbs0143

<220>
<221> misc_feature
<222> (1)..(15)

<400> 168
attttcagat aattg                                                        15


<210> 169
<211> 15
<212> DNA
<213> Artificial Sequence

<220>
<223> Description of Artificial Sequence: presence of
      the putative CodY box in gbs0604

<220>
<221> misc_feature
<222> (1)..(15)

<400> 169
atttttagaa aatta                                                        15


<210> 170
<211> 15
<212> DNA
<213> Artificial Sequence

<220>
<223> Description of Artificial Sequence: presence of
      the putative CodY box in dnaG

<220>
<221> misc_feature
<222> (1)..(15)

<400> 170
attttctgaa taatt                                                        15

<210> 171
<211> 15
<212> DNA
<213> Artificial Sequence

<220>
<223> Description of Artificial Sequence: presence of
      the putative CodY box in ftsA

<220>
<221> misc_feature
<222> (1)..(15)

<400> 171
attttctgaa taatt                                                        15


<210> 172
<211> 15
<212> DNA
<213> Artificial Sequence

<220>
<223> Description of Artificial Sequence: presence of
      the putative CodY box in gbs1259

<220>
<221> misc_feature
<222> (1)..(15)

<400> 172
attttcagga tattt                                                        15


<210> 173
<211> 288
<212> DNA
<213> Artificial Sequence

<220>
<223> Description of Artificial Sequence: upstream
      sequence encompassing the opp-pepO1 operon

<220>
<221> misc_feature
<222> (1)..(288)

<400> 173
ctgatatcaa taaagaataa aaagtccaaa acagagcagc atgaaaaata aaacaataat 60
aaaagcagtt tttagtatga ttactgcttt tattatttcc tccaaaactt ttgctttacc 120
tttatttcgc gtaatgttca gaaaattcat gaacatacct aaaatagtaa atttttgcaa 180
atatgcagaa aaagtagtat acttttatta agtctattta gaaagatttt attgaggtaa 240
atatggaaag tgaaaatatt ttggaagcaa aacaagtgag tgttgctt             288


<210> 174
<211> 15
<212> PRT
<213> Artificial Sequence

<220>

<223> Description of Artificial Sequence: part of oppD

<220>
<221> SITE
<222> (1)..(15)

<400> 174
Met Glu Ser Glu Asn Ile Leu Glu Ala Lys Gln Val Ser Val Ala
1               5                   10                  15


<210> 175
<211> 29
<212> DNA
<213> Artificial Sequence

<220>
<223> Description of Artificial Sequence: WT

<220>
<221> misc_feature
<222> (1)..(29)

<400> 175
cgtaatgttc agaaaattca tgaacatac                                    29


<210> 176
<211> 29
<212> DNA
<213> Artificial Sequence

<220>
<223> Description of Artificial Sequence: Opp15 (a)

<220>
<221> misc_feature
<222> (1)..(29)

<400> 176
cgtaatatta agaaaattca tgaacatac                                    29


<210> 177
<211> 29
<212> DNA
<213> Artificial Sequence

<220>
<223> Description of Artificial Sequence: Opp15 (b)

<220>
<221> misc_feature
<222> (1)..(29)

<400> 177
cgtactgtgc cgaaaattca tgaacatac                                    29


<210> 178
<211> 30

<212> DNA
<213> Artificial Sequence

<220>
<223> Description of Artificial Sequence: WT

<220>
<221> misc_feature
<222> (1)..(30)

<400> 178
cgtaatgttc agaaaattca tgaacatacc                                        30


<210> 179
<211> 30
<212> DNA
<213> Artificial Sequence

<220>
<223> Description of Artificial Sequence: MUT2

<220>
<221> misc_feature
<222> (1)..(30)

<400> 179
cgtaatgttc tgaaaattca tgaacatacc                                        30


<210> 180
<211> 30
<212> DNA
<213> Artificial Sequence

<220>
<223> Description of Artificial Sequence: MUTF

<220>
<221> misc_feature
<222> (1)..(30)

<400> 180
cgtaatgttc agaaaattca tggacatacc                                        30


<210> 181
<211> 30
<212> DNA
<213> Artificial Sequence

<220>
<223> Description of Artificial Sequence: MUT4

<220>
<221> misc_feature
<222> (1)..(30)

<400> 181
cgtaatgttc agaaaattca tgagcatacc                                        30

<210> 182
<211> 30
<212> DNA
<213> Artificial Sequence

<220>
<223> Description of Artificial Sequence: MUT3

<220>
<221> misc_feature
<222> (1)..(30)

<400> 182
cgtagtgttc agaaaattca tgaacatacc                                        30


<210> 183
<211> 30
<212> DNA
<213> Artificial Sequence

<220>
<223> Description of Artificial Sequence: MUT10

<220>
<221> misc_feature
<222> (1)..(30)

<400> 183
cgtaatgtcc agaaaattca tgaacatacc                                        30


<210> 184
<211> 30
<212> DNA
<213> Artificial Sequence

<220>
<223> Description of Artificial Sequence: MUT16

<220>
<221> misc_feature
<222> (1)..(30)

<400> 184
cgtaatgttc ggaaaattca tgaacacacc                                        30


<210> 185
<211> 26
<212> DNA
<213> Artificial Sequence

<220>
<223> Description of Artificial Sequence: motif as
      observed in L. lactis

<220>
<221> misc_feature
<222> (1)..(26)

<223> /note="Sequence wherein n can be any nucleotide"

<400> 185
anaattttct ganaaatnna tnanta                                          26


<210> 186
<211> 26
<212> DNA
<213> Artificial Sequence

<220>
<223> Description of Artificial Sequence: consensus
      motif as observed in L. lactis

<220>
<221> misc_feature
<222> (1)..(26)
<223> /note="Sequence wherein n can be any nucleotide, w
      can be t or a, r can be a or g, d can not be c and
      h can not be g"

<400> 186
whaattdtcw gahaawtnnr wnadww                                          26


<210> 187
<211> 15
<212> DNA
<213> Artificial Sequence

<220>
<223> Description of Artificial Sequence: consensus
      motif as observed in B. subtilis

<220>
<221> misc_feature
<222> (1)..(15)
<223> /note="Sequence wherein w can be t or a and d can
      not be c"

<400> 187
awttdtcaga awwwt                                                      15


<210> 188
<211> 14
<212> DNA
<213> Artificial Sequence

<220>
<223> Description of Artificial Sequence: motif as
      observed in B. subtilis

<220>
<221> misc_feature
<222> (1)..(14)
<223> /note="Sequence wherein n can be any nucleotide"

<400> 188
attntcagaa aatt                                                       14

<210> 189
<211> 225
<212> DNA
<213> Lactococcus lactis Wg2

<220>
<221> promoter
<222> (1)..(225)
<223> /note="prtP/prtM promoter region"

<400> 189
tgctaaaaat ttcaaaacat ctatagtctg taaacggcta aataataacg ctaaaagtta 60
atttacagat aaaaaaatta atagaagatt aaaattttcg ttgaatttgt tcttcaatag 120
tatataatat aatagtatat aatattatat aatataatct taactacatc aagcgtaggg 180
tttgatttgg ttatgaaact tttggaaagt ggaggatatt ggatg 225


<210> 190
<211> 230
<212> DNA
<213> Lactococcus lactis SK11

<220>
<221> promoter
<222> (1)..(230)
<223> /note="prtP/prtM promoter region"

<400> 190
tgctaaaaat ttcaaaacat ctatagtctg taaacggcta aataataacg ctaaaagtta 60
atttacagat aaaaaaatta atagaagatt aaaattttcg ttgaatttgt tcttcaatag 120
tatataatat aatagtatat aatattatat tatataatat aatcttaact acatcaagcg 180
taggctttga tttggttatg aaacttttgg aaagtggagg atattggatg 230


<210> 191
<211> 230
<212> DNA
<213> Lactococcus lactis E8

<220>
<221> promoter
<222> (1)..(230)
<223> /note="prtP/prtM promoter region"

<400> 191
tgctaaaaat ttcaaaacat ctatagtctg taaacggcta aataataacg ctaaaagtta 60
atttacagat aaaaaaatta atagaagatt aaaattttcg ttgaatttat tcttcaatag 120
tatataatat aatattatat aatattatat tatataatat aatcttaact acatcaagcg 180
tagggtttga tttggttatg aaacttttgg aaagtggagg atattggatg 230


<210> 192
<211> 230
<212> DNA
<213> Lactococcus lactis BGMN1-5

<220>
<221> promoter
<222> (1)..(230)

<223> /note="prtP/prtM promoter region"

<400> 192
tgctaaaaat ttcaaaacat ctatagtctg taaacggcta aataataacg ctaaaagtta 60
atttacagat aaaaaaatta atagaagatt aaaattttag ttgaatttgt tctttaatag 120
tatataatat aatagtatat actattatat tatatactat tatattaact acatcaagcg 180
tacattttga tttggttatg aaacttttgg aaagtggagg gtattggatg 230

## Description

The invention relates to the field of biochemistry, molecular biology and food production. More in particular, the invention relates to methods and means for regulating gene expression. Even more in particular, the invention relates to CodY target sequences.

The Gram-positive lactic acid bacterium *Lactococcus lactis* is an important microorganism in dairy food production. It is part of many starter cultures used in cheese manufacturing, where its function is to degrade the milk protein casein into small peptides and amino acids (Kok and Vos, 1993). *L. lactis*, like other lactic acid bacteria, is a multiple amino acid auxotroph. It has a complex proteolytic system to break down the major milk protein casein into small peptides and free amino acids that are necessary for growth in this medium (Kunji *et al*., 1996, Christensen *et al*., 1999). Initial breakdown of casein is carried out by the extracellular cell wall-bound serine proteinase PrtP. Several lactococcal *prtP* genes have been cloned and sequenced (Kok *et al*., 1985, Kok *et al*., 1988, de Vos *et al*., 1989, Kiwaki *et al*., 1989). Although they are over 98% identical on the amino acid sequence level, the proteinases can have quite different caseinolytic specificities (Visser *et al*., 1986). For the production of an active proteinase, the product of *prtM*, a gene that is in a back-to-back orientation with *prtP*, is required. The so-called maturase PrtM plays a role as an extracellular chaperone, inducing the pro-proteinase to adopt a conformation in which it is able to autoproteolytically cleave off its pro-region (Kok, 1990, Haandrikman, 1990). Peptides that are produced by the proteinase can be internalized by either one of three different transport systems. Oligopeptides are taken up by Opp, while DtpT and DtpP transport di- and three-peptides respectively (Tynkkynen *et al*., 1993, Foucaud *et al*., 1995). Intracellularly, the peptides are further hydrolyzed into smaller peptides and amino acids by the action of over 15 different peptidases (Kunji *et al*., 1996, Christensen *et al*., 1999).

Proteinase and maturase production is inhibited in peptide-rich medium (e.g. containing casitone, a tryptic digest of casein) in a number of lactococcal strains (Exterkate, 1985, Laan *et al*., 1993, Marugg *et al*., 1995, Miladinov *et al*., 2001). As PrtP expression is not down-regulated in strains that lack the di- and tripeptide transporter DtpT, it was hypothesized that the internal concentration of small (di-tri) peptides, or amino acids derived thereof, are important in the regulation of proteinase production (Marugg *et al*., 1995). The genetic information for proteinase regulation was shown to be present on a 90-bp subfragment of the *prtP*/*prtM* intergenic region encompassing the transcription start sites of both genes (Marugg *et al*., 1996). Disruption of an inverted repeat that is present in this region resulted in derepression of the *prtP* and *prtM* promoters in medium with a high peptide concentration.

The expression of genes of other components of the proteolytic system of *L. lactis* is also affected by medium composition. The expression of OppA, DtpT and DtpP is increased when cells are grown in medium with a low peptide concentration (Detmers *et al*., 1998, Kunji *et al*., 1996, Foucaud *et al*., 1995). Moreover, the expression of the peptidases PepX and PepN in *L. lactis* MG1363 was shown to be regulated in a similar way (Meijer *et al*., 1996). Promoters of *pepC*, *pepN, pepO1*, and *pepO2* were also reported to be more active in medium with amino acids than in peptide-rich medium (Guedon *et al*., 2001a). In the same study, the *prtP* promoter was shown to be subject of a similar regulatory circuit.

Recently, a pleiotropic regulator, CodY, has been identified in *L. lactis* MG1363 that represses several genes involved in the processes mentioned above (Guedon *et al*., 2001b). CodY, was first identified in the Gram-positive bacterium *Bacillus subtilis,* in which it also serves as a repressor of several genes involved in proteolysis (Serror and Sonenshein, 1996b; Serror and Sonenshein, 1996a). In *B. subtilis,* the activity of CodY is dependent on intracellular GTP levels, thereby sensing the energy state of the cell (Ratnayake-Lecamwasam *et al*., 2001). For *L. lactis* it was shown that the repression by CodY is relieved upon a decrease in the intracellular pool of the branched chain amino acids (BCAA's) Leu, Iso and Val (Guedon *et al*., 2001b).

Like its *B. subtilis* counterpart, CodY of *L. lactis* contains a C-terminal helix-turn-helix DNA binding motif. In *B. subtilis* it has been shown that the protein is able to bind to sequences overlapping the -35 and -10 sequences of its target promoters (Serror and Sonenshein, 1996b; Fisher, Rohrer, and Ferson, 1996).

Herein we show that CodY represses its target genes by binding to specific DNA sequences upstream of the respective genes. A conserved target site was identified by analyzing upstream sequences of derepressed genes in a delta *codY L. lactis* MG1363 derivative, as identified in a DNA micro-array study. The present application furthermore discloses CodY target sequences from other gram-positive bacteria, like *B. subtilis* and *Streptococcus*.

Hence, the invention provides CodY target sequences that may be used in different applications to repress or derepress gene expression.

In a first embodiment, the invention provides a method for regulating the expression of a gene of interest in a host cell that comprises a CodY-like protein comprising providing said cell with a gene of interest in operable linkage with a promoter and at least one CodY target sequence.

Regulation of gene expression is a very desirable characteristic of gene expression systems. For example, when one would like to express a protein that is toxic for the used host cell, preferably a gene encoding said protein is under the control of a regulator which can be switched on or off at will. Typically, for production of such a protein, expression of the corresponding gene is suppressed until enough biomass has been obtained and then expression of said gene is obtained for example by providing an inducer. However, also expression of non-toxic proteins is preferably regulated by an induction system. Examples of these kinds of expression systems are well known in the art and hence no further elaboration on this subject is necessary. In a method according to the invention a CodY-like protein and at least one CodY target sequence, control expression of a gene of interest. Binding of a CodY-like protein to said at least one CodY target sequence results in repression of expression of the gene of interest that is under control of said CodY target sequence. In the absence of (sufficient) CodY-like protein or in the presence of non-functional (i.e. non-binding) CodY-like protein, said gene of interest is expressed. Hence, the invention provides a way for regulating gene expression. As CodY-like proteins are typically found in gram-positive bacteria, for example lactic acid bacteria, the invention preferably provides a method for regulation gene expression in gram-positive bacteria. However, it is clear to the person skilled in the art that necessary components of the method according to the invention, i.e. a CodY-like protein and a CodY target sequence may easily be transferred to for example a gram-negative bacterium.

CodY proteins show a large amount of homology in gram-positive bacteria with a low G+C content. A Blast search with the CodY amino acid sequence of *L. lactis* shows homology with *Bacillus subtilis, Bacillus anthracis*, *Bacillus halodurans, Bacillus stearothermophilus*, *Clostridium acetobutylicum*, *Clostridium difficile, Enterococcus faecalis, Staphylococcus aureus*, *Streptococcus mutans, Streptococcus pneumoniae* and *Streptococcus pyogenes*. Moreover, these CodY proteins all comprise a DNA binding motif, preferably a helix-turn-helix DNA binding motif. It is furthermore shown that CodY proteins comprise putative GTP binding motifs as summarised in Table 1. Furthermore, binding of these CodY proteins to their target sequences is typically under the influence of the energy level of the cell (GTP) or the intracellular pool of branched chain amino acids or the nutritional value of the medium (nitrogen source like casitone).

A CodY-like protein is typically a CodY protein or a functional equivalent and/or a functional fragment thereof, obtained/derived from a gram-positive bacterium, which CodY protein comprises the above outlined characteristics, i.e. capable of binding to a (consensus) CodY target sequence and sensitive to a change in the energy level of the cell, the intracellular pool of branched amino acids or the medium composition. Examples of CodY proteins are the CodY proteins from *Lactococcus lactis* (Guedon et al, 2001b)) or *Bacillus subtilis* (Serror and Sonenshein, 1996b; Serror and Sonenshein, 1996a). It is clear that for example a CodY protein from *L. lactis* can be modified without significantly changing the above outlined characteristics, for example, by introducing point mutations or (small) deletions. Hence, a CodY-like protein is a Cod protein obtained from a gram-positive bacterium such as *Bacillus subtilis, Bacillus anthracis, Bacillus halodurans, Bacillus stearothermophilus, Clostridium acetobutylicum, Clostridium difficile*, *Enterococcus faecalis, Staphylococcus aureus, Streptococcus mutans*, *Streptococcus pneumoniae, Streptococcus pyogenes*, and *Lactococcus lactis*, possibly comprising mutations which do not interfere significantly with for example the binding of said CodY-like protein to a CodY target sequence and furthermore is sensitive to the energy state of a cell, the intracellular pool of branched amino acids or the medium composition.

The location of the CodY target sequence with regard to the promoter sequence is flexible. The CodY target sequence may be either located upstream, downstream or overlapping with regard to the -35 and -10 sequences. Furthermore, at least one CodY target sequence is used in a method according to the invention. As disclosed herein within the experimental part, an increase in the number of CodY target sequences results in a more pronounced regulation of expression and hence introduction of more than one CodY target sequence is useful depending on, for example, the characteristics of the gene of interest.

The promoter used in a method according to the invention is preferably a promoter that is functional in the used host cell. For example, a promoter that is functional in a gram-positive bacterium is used, in operable linkage with a CodY target sequence and a gene of interest, for regulating expression of said gene of interest in a gram-positive bacterium. The prior art provides a large amount of promoter sequences, both from gram-positive as well as gram-negative bacteria, and hence no further elaboration on this item is necessary.

In a preferred embodiment, said promoter and/or said CodY target sequence is heterologous with regard to said gene of interest. In yet another preferred embodiment, said CodY target sequence is heterologous with regard to said promoter. Hence, the invention preferably makes use of combinations in which at least one component (i.e. promoter sequence or gene of interest or CodY target sequence) is different when compared to wild type/natural situation. The gene of interest may be an endogenous gene or a heterologous gene. For an endogenous gene that is already in operable linkage with a promoter, only at least one CodY target sequence has to be introduced (in operable linkage with said promoter and said gene). After introduction of said at least one CodY target sequence, expression of said endogenous gene will, in the presence/absence of CodY-like protein, be repressed/derepressed and hence expression of said endogenous gene is regulated. Furthermore, it is within the scope of the present application to introduce an extra copy of an endogenous gene in operable linkage with its own promoter and/or at least one CodY target sequence or with another promoter and/or at least one CodY target sequence. Hence, at least two copies of said endogenous gene are then present. A heterologous gene in operable linkage with a promoter and at least one CodY sequence may for example be introduced via a plasmid. However, it also possible to only introduce said gene of interest and further provide said gene of interest with the necessary means for homologous recombination to an endogenous gene that is under control of a CodY target sequence. In this way an endogenous gene is replaced by another gene, which is, then under control of a CodY target sequence.

The introduction of new and/or extra genetic information into a host cell may be accomplished by methods known in the art, for example by electroporation, protoplast transformation, transfection, transduction or any other known method.

Any sequence can be used as sequence of interest. Preferably, said sequence enables the production of a protein of interest not present as such or present in a (too) low concentration, in said cell. For example, a sequence/open reading frame (ORF) specifying an enzyme (protease or peptidase), a vitamin or an anti-microbial peptide is used. Preferably, said gene of interest is a gene from a gram-positive bacterium. Even more preferably, said gene of interest is a gene from a lactic acid bacterium, like *Lactococcus, Lactobacillus*, *Streptococcus, Leuconostoc, Pediococcus, Bifidobacterium, Carnobacterium* or *Propionibacterium*. An example of a gene of interest is a gene that encodes a protease or a peptidase or an anti-microbial peptide or a vitamin. Other examples of gene products include, but is not limited to, hydrolytic enzymes selected from proteases such as chymosin, peptidases including endopeptidases, lipases, nucleases and carbohydrases; lytic enzymes such as lysozyme or phage lysins; flavour enhacing substances; bacteriocins including nisin, pediocin and bavaracin; amino acids; organic acids; and pharmacologically active substances.

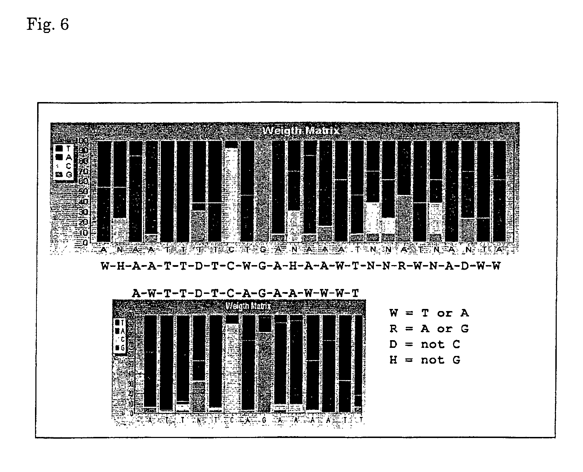

In a preferred embodiment the invention provides a method for regulating the expression of a gene of interest in a host cell that comprises a CodY-like protein comprising providing said cell with a gene of interest in operable linkage with a promoter and at least one CodY target sequence, wherein said CodY target sequence comprises a sequence as depicted in the upper part of Figure 6, or a functional equivalent and/or a functional fragment thereof. The upper part of Figure 6 discloses the consensus CodY target sequence. Moreover, the invention provides in Table 4 multiple examples of *L*. *lactis* CodY target sequences that provide non-limiting examples of combinations of W, R, D and H as depicted in Figure 6. Until the present patent application, no (consensus) sequence for CodY binding was disclosed. Now that the consensus sequence and some of its variants are disclosed herein (see upper part of Figure 6 and Table 4) a person skilled in the art is very well capable of obtaining a functional equivalent and/or a functional fragment of said consensus sequence. A functional equivalent and/or a functional fragment must still be capable of binding a CodY-like protein. A functional equivalent is for example obtained by screening other bacteria for the presence of the herein disclosed CodY target sequences. For example, the present inventors have identified CodY target sequences in *Bacillus subtilis, Streptococcus pneumoniae* and *Streptococcus agalacticiae*, as disclosed herein within Figure 6 lower part, Table 5, 6, 7 or 8. The lower part of Figure 6 discloses the CodY target consensus sequence in *B. subtilis* and Table 5 and 6 show multiple examples of the typical CodY target sequences. Table 7 and 8 disclose multiple examples of *Streptococus* CodY target sequences. It is clear that point mutation and deletion studies lead to further functional equivalents and/or functional fragments and hence these also within the scope of the present patent application.

Based on the herein disclosed (consensus) CodY target sequences it is furthermore possible to construct for example constructs comprising two or more (identical or different) CodY-like target sequences. In this way a more stringent regulation of expression is obtained. For example a gene of interest in operable linkage with a promoter and two (identical or different) CodY target sequence is used to obtain more stringent control of expression. However, it is also possible to introduce a construct that comprises multiple CodY target sequences (with or without a promoter and/or a gene of interest) in a cell that comprises CodY regulated genes and hence a competitive binding of CodY to said construct that comprises multiple CodY target sequences and binding to a CodY target sequence in operable linkage with a gene of interest and a promoter takes place. In this way a gene of interest is derepresses and said gene of interest is expressed.

The method according to the invention allows both active as well as inactive/passive regulation of gene expression of a gene of interest. Said regulation is preferably based on influencing the binding between a CodY-like protein and at least one CodY target sequence. An example of passive/indirect/inactive regulation is a gene of interest in operable linkage with a promoter and a CodY target sequence that is introduced into a host cell that comprises CodY-like protein. During exponential growth of said host cell said CodY-like protein binds to said CodY target sequence and hence expression of said gene of interest is repressed. After the exponential phase, said CodY-like protein will release from said CodY target sequence and hence expression of said gene of interest is induced. Such an approach is extremely useful in cases in which one would like to have expression of a gene of interest after exponential growth. Active regulation of gene expression of a gene in operable linkage with a promoter and a at least one CodY target sequence is for example obtained by regulating binding of a CodY-like protein and a CodY target sequence by subjecting said cell to a change in a growth condition, preferably to a growth limiting condition like a limited availability of a nitrogen source. In case, a CodY-like protein or a functional fragment and/or a functional derivative thereof of *L. lactis* is used, means that result in a decrease in the intracellular pool of the branched amino acids Leu, Iso and Val results in relief of CodY repression. The CodY protein of *B. subtilis* is for example actively regulated by a means that influence the level of GTP in a host cell. Hence, actively subjecting a CodY-like protein comprising host cell that further comprises a gene of interest in operable linkage with a promoter and at least one CodY target sequence to a medium with a limited availability of a nitrogen source results in derepression and hence expression of said gene of interest.

In a preferred embodiment, the invention provides a method for regulating the expression of a gene of interest in a host cell that comprises a CodY-like protein comprising providing said cell with a gene of interest in operable linkage with a promoter and at least one CodY target sequence, wherein said host cell is a cell from a food production species and even more preferably a diary food production species. Preferably, said species is selected from the gram-positive species and even more preferably said species is a lactic acid bacterium such as *Lactococcus* or *Lactobacillus* or *Streptococcus* or *Leuconostoc* or *Pediococcus* or *Bifidobacterium* or *Carnobacterium*. An example of a gram-positive, non lactic acid bacterium is *Propionibacterium*. Amongst others, these species are used in the production of food, for example in a fermentation step for the production of a dairy product. Hence, by providing these species with a gene of interest under the control of a promoter and at least one CodY target sequence and either indirectly/passively or directly/actively influencing the binding between a CodY-like protein and its target sequence results in repression or derepression (i.e regulation) of gene expression of said gene of interest. This may be used for the metabolic engineering of various catabolic pathways by a rerouting strategy consisting of the controlled overproduction and/or disruption of genes. For example, genes of which the products, directly or indirectly, are involved in the production of compounds that are involved in the formation of off-flavours during exponential growth during a (dairy) food production, are repressed by providing said genes with a CodY target sequence. Food or dairy food production species in which said genes are under the control of a CodY target sequence, will produce less (or preferably no) off-flavours and hence these production processes are improved. In an analogous way it is also possible to induce expression of a gene of interest after the exponential growth and hence provide said species with altered flavour formation, altered cell lysis capabilities or induce production of health promoting substances (such as vitamins) or provide said species with means to at least in part prevent acidification of the same or another species. For the latter possibilities, a gene of interest (for example a gene involved in cell lysis or flavour formation or a gene encoding a vitamin) is placed under the control of a promoter and at least one CodY target sequence and after the end of exponential growth, CodY-like protein will be released from said CodY target sequence and hence expression of said gene is induced. Furthermore, CodY-like proteins are released from their target sequences by providing cells with for example synthetic CodY targets. Said CodY target may be added to the medium, taken up by the cells (for example *B. subtilis*) and the CodY-like proteins are released from their targets and will bind to the synthetic CodY targets.

Now that the invention discloses multiple (consensus) CodY target sequences it is furthermore part of the invention to modify endogenous CodY sequences, to for example increase or decrease (or more generally alter) binding of CodY to one of its endogenous target sequences and hence production of an endogenous gene that is under control of the CodY/CodY target sequence regulation may be altered. For example, proteins which expression is under the control of CodY (and hence are not or hardly not produced during the exponential growth phase) but whose product provides advantageous uses when present during exponential growth may now be amended such that binding of CodY to said CodY target sequence is not or hardly not possible under the exponential growth phase conditions. Hence, said protein is expressed during exponential growth and advantage is taken of the properties of said expressed protein.

In case a host cell does not comprise or does not comprise enough CodY-like protein or comprises non-functional (i.e. CodY protein that is not capable of binding to a CodY target sequence) CodY-like protein, the invention furthermore provides a method for regulating the expression of a gene of interest in a host cell that comprises a CodY-like protein comprising providing said cell with a gene of interest in operable linkage with a promoter and at least one CodY target sequence wherein said host cell is further provided with a nucleic acid encoding a CodY-like protein. Examples of CodY sequences are already outlined above and hence no further details are provided. In case a host cell does not comprise/express enough CodY-like protein said cell may be provided with either endogenous and/or heterologous CodY-like protein.

In yet another preferred embodiment, the invention provides an isolated or recombinant nucleic acid that comprises at least one CodY target sequence or a functional fragment and/or a functional equivalent thereof. It is clear from Figure 6 (lower and upper part) and Tables 4 to 8, that the length of said nucleic acid generally comprises 15 to 30 nucleotides. A functional fragment and/or a functional equivalent thereof is defined as a fragment and/or equivalent that is capable of binding a CodY-like protein. A functional fragment is for example obtained by introducing an N-, C- or internal deletion. Examples of suitable CodY target sequences are provided herein (Fig. 6 and Tables 4 to 8). A nucleic acid that comprises multiple (either identical or different) CodY target sequences is also included herein. Furthermore, artificial or synthetic CodY target sequences are also included herein.

In a preferred embodiment said isolated or recombinant nucleic acid that comprises at least one CodY target sequence or a functional fragment and/or a functional equivalent thereof, further comprises a promoter sequence and/or a promoter sequence in operable linkage with a gene of interest.

In case a host cell does not comprise or does not comprise enough endogenous CodY-like or comprises non-functional CodY protein, the invention furthermore provides an isolated or recombinant nucleic acid that comprises at least one CodY target sequence or a functional fragment and/or a functional equivalent thereof wherein said nucleic acid further comprises a gene encoding a CodY-like protein or a functional fragment and/or a functional equivalent thereof.

In a preferred embodiment, said promoter and/or said at least one CodY target sequence is heterologous with regard to said gene of interest and in another preferred embodiment, said CodY target sequence is heterologous with regard to said promoter.

Again, as already outlined above, said gene of interest may either be an endogenous and/or a heterologous gene. Preferably, said gene of interest is a gene from a gram-positive bacterium, such as a gene from a lactic acid bacterium for example *Lactococcus* or *Lactobacillus* or *Streptococcus* or *Leuconostoc* or *Pediococcus* or *Bifidobacterium* or *Carnobacterium*. An example of a gram-positive, non lactic acid bacterium is *Propionibacterium*.

A gene of interest may be any gene, preferably said gene of interest encodes a protease or a peptidase or an anti-microbial peptide or a vitamin. Other suitable examples include hydrolytic enzymes selected from proteases such as chymosin, peptidases including endopeptidases, lipases, nucleases and carbohydrases; lytic enzymes such as lysozyme or phage lysins; flavour enhacing substances; bacteriocins including nisin, pediocin and bavaracin; amino acids; organic acids; and pharmacologically active substances.

In a preferred embodiment, the invention provides an isolated or recombinant nucleic acid that comprises at least one CodY target sequence or a functional fragment and/or a functional equivalent thereof, wherein said CodY target sequence comprises a sequence as depicted in Figure 6 or a functional equivalent and/or a functional fragment thereof. The upper part of Figure 6 discloses a consensus CodY target sequence. Moreover, the invention provides in Table 4 multiple examples of *L. lactis* CodY target sequences that provide non-limiting examples of combinations of W, R, D and H as depicted in Figure 6. Until the present patent application, no (consensus) sequence for CodY binding was disclosed. Now that the consensus sequence and some of its variants are disclosed herein (see upper part of Figure 6 and Table 4) a person skilled in the art is very well capable of obtaining a functional equivalent and/or a functional fragment of said consensus sequence. A functional equivalent and/or a functional fragment must still be capable of binding a CodY-like protein. A functional equivalent is for example obtained by screening other bacteria for the presence of the herein disclosed CodY target sequences. For example, the present inventors have identified CodY target sequences in *Bacillus subtilis, Streptococcus pneumoniae* and *Streptococcus agalacticiae*, as disclosed herein within Figure 6 lower part, Table 5, 6, 7 or 8. The lower part of Figure 6 discloses the CodY target consensus sequence in *B.* *subtilis* and Table 5 and 6 show multiple examples of the typical CodY target sequences. Table 7 and 8 disclose multiple examples of *Streptococus* CodY target sequences. It is clear that point mutation and deletion studies lead to further functional equivalents and/or functional fragments and hence these also within the scope of the present patent application.

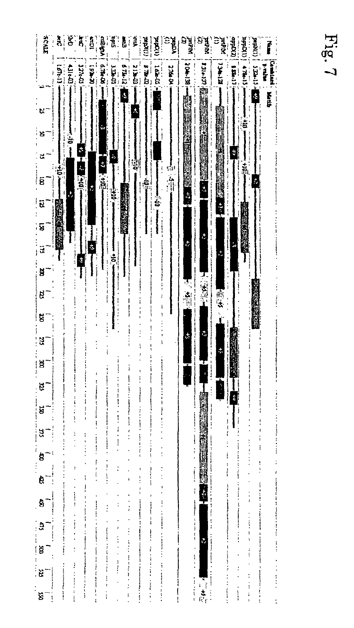

Moreover, the present inventors have identified alternative sequences in the upstream region of CodY regulated genes that may also be included in a method for regulating the expression of a gene of interest in a host cell that comprises a CodY-like protein (Figure 7). These sequences may also be used in a method and/or nucleic acid according to the invention.

In another embodiment the invention provides a vector comprising a nucleic acid as described above. Said vector may further be provided with means for homologous recombination. With these means said at least one CodY target sequence and/or a gene of interest and/or a promoter and/or a gene encoding CodY-like protein may be integrated into the genome of a cell and hence a more stable situation may be obtained. In yet another embodiment, the invention provides a gene delivery vehicle comprising a nucleic acid or a vector according to the invention. Gene delivery vehicles are well known in the art and hence no further details are provided on this subject matter.

In a further embodiment the invention provides a host cell that comprises a nucleic acid, a vector or a gene delivery vehicle according to the invention. Preferably, said host cell is a cell from a food production species and even more preferably said host cell is a cell from a dairy food production species. Non-limiting examples of said species are gram positive lactic acid bacteria such as *Lactococcus* or *Lactobacillus* or *Streptococcus* or *Leuconostoc* or *Pediococcus* or *Bifidobacterium* or *Carnobacterium*. An example of a gram positive, non lactic acid species is *Propionibacterium*.

In another embodiment, the invention provides use of at least one CodY target sequence for regulating the expression of a gene of interest. Preferably, said at least one CodY target sequence is selected from Figure 6 or Tables 4 to 8. For example, the use of at least one CodY target sequence in operable linkage with a promoter and a gene of interest in a Cod-like protein comprising host cell result, for example under exponential growth in repression of expression of said gene of interest. After exponential growth, CodY protein will be released from its target, resulting in depression of expression of said gene of interest.

In yet another embodiment the invention provides a method for producing a food product comprising a nucleic acid or a vector or a gene delivery vehicle or a host cell as described above. Preferably said food product is a diary food product. As a non-limiting example, the use of a host cell is described in more detail. A lot of (diary) food production processes involve the use of a (fermenting) host cell. These host cells may now be manipulated with regard their protein products. For example, genes of which the products, directly or indirectly, are involved in the production of compounds that are involved in the formation of off-flavours during exponential growth during a (dairy) food production, are repressed by providing said genes with a CodY target sequence. Food or dairy food production species in which said genes are under the control of a CodY target sequence, will produce less (or preferably no (detectable)) off-flavours during exponential growth and hence these production processes are altered. In an analogous way it is also possible to induce expression of a gene of interest after the exponential growth of said host cell and hence provide said species with altered flavour formation, altered cell lysis capabilities or induce production of health promoting substances (such as vitamins) or provide said species with means to prevent acidification of the same or another species. For the latter possibilities, a gene of interest (for example a gene involved in cell lysis or a gene involved in flavour formation or a gene encoding a vitamin) is placed under the control of a promoter and at least one CodY target sequence and after the end of exponential growth, CodY-like protein will be released from said CodY target sequence and hence expression of said gene is induced.

With regard to a fluid dairy product a method to at least in part decrease lysis of bacteria and/or acidification after production of said fluid diary product, i.e. after exponential growth of the used bacteria, is very advantageous with regard to the shelf life of said fluid dairy product. First, genes that are capable of at least in part preventing lysis and/or acidification are identified. After identification, such genes are placed under the regulation of at least one CodY target sequence. Said genes are expressed and hence lysis and/or acidification is at least in part prevented (and more preferably completely inhibited) and hence the shelf life of said product is increased.

Moreover, in case integration of said nucleic acid or vector is desired, use may be made of food grade integration techniques (for example see Leenhouts, 1995, herein incorporated by reference)

Preferably, the invention provides a method for producing a (dairy) food product comprising a nucleic acid or a vector or a gene delivery vehicle or a host cell as described herein, wherein said dairy product is a cheese or a fermented milk product. The production of a lot of dairy products involves fermentation of lactic acid bacteria and hence the application of modified host cells as described herein are particularly advantageous. The term "dairy product" include but is not limited to cheese, fluid dairy products like milk and yoghurt, fermented milk product, ice cream, butter, buttermilk, margarine and milk powder.

In another embodiment the invention provides food or a dairy food, such as a cheese or a fermented milk product, obtainable by a method according to the invention. Such a product comprises for example a different or extra taste or comprises specific compounds/structures (such as health improving compounds as vitamins) not present in food or dairy food product not obtained according to a method of the invention. Food or dairy food products prepared by a method according to the invention may comprise different amounts or different kinds of enzymes, peptides, amino acids, flavour enhancing or pharmacologically active substances or organic acids.

In yet another embodiment, the invention provides a method for at least in part preventing the formation of off-flavours during a process for the production of a (dairy) food product, comprising providing at least one CodY target sequence in operable linkage with a gene which product is, directly or indirectly, involved in the formation of off-flavours.

Now that the present inventors have disclosed CodY target sequences. This finding provide interesting leads in the fight against food-spoilers and pathogens.

PpmA is a recently identified pneumococcal protein with significant sequence homology to the proteinase maturation protein (PrtM) of lactic acid bacteria. PrtM is a *trans*-acting protein involved in the processing of precursors of serine protease PrtP into active enzymes and belongs to the family of peptidyl-prolyl cis/trans isomerases. These enzymes are thought to assist in protein folding by catalyzing the *cis*/*trans* isomerization of the petidyl-prolyl bonds in peptides and proteins. The pneumococcal proteins(s) that is activated by PpmA is currently unknown. PpmA of *Streptococcus pneumoniae* was demonstrated to be involved in virulence. Inactivation of *ppmA* significantly reduced the virulence of strain D39 for mice as judged by the survival time after intranasal challenge. The present inventors identified a CodY target sequence upstream of *ppmA* (Table 7) indicating that the expression of this gene probably is under the control of CodY. Hence, the invention provides a method for regulating the expression of *ppmA*.

Spore-forming bacteria (e.g. *Bacillus*) can cause serious problems in industrial food fermentations as the spores can survive most processing conditions. In *Bacillus subtilis* it was shown that the target genes of CodY generally encode proteins useful to the cell in adapting to poor nutritional conditions, but also include several genes whose expression is critical to the acquisition of genetic competence and the initiation of sporulation. The present invention provides a method for influencing the expression of these genes.

In *Clostridium difficile,* the synthesis of two toxin proteins responsible for antibiotic-associated colitis and pseudomembranous colitis were shown to be dependent on a RNA polymerase sigma factor TxeR (Sonenshein, unpublished). Both TxeR and the toxin proteins were not synthesized in exponential phase cells, probably due to the action of CodY as it was shown to bind to the toxin regulatory region.

The invention will now be illustrated by means of the following, non-limiting examples.

### EXPERIMENTAL PART MATERIALS AND METHODS

### Bacterial strains, plasmids and growth conditions

The strains and plasmids used in this study are listed in Table 9. *Escherichia coli* was grown in TY medium at 37°C with vigorous agitation or on TY medium solidified with 1.5% agar, containing 100 µg of erythromycin per ml when needed. 5-bromo-4-chloro-3-indolyl-β-D-galactopyranoside (X-gal) was used at a final concentration of 40 µg/ml. *L. lactis* was grown at 30°C in M17 broth or on M17 medium solidified with 1.5% agar, supplemented with 0.5% glucose. When needed, erythromycin, chloramphenicol and X-gal were added at final concentrations of 5 µg, 5 µg and 80 µg per ml, respectively. Chemically defined medium (CDM) was prepared according to Poolman and Koning (1988).

### DNA preparation, molecular cloning and transformation

Routine DNA manipulations were performed as described by Sambrook *et al*.(1989). Total chromosomal DNA from *L. lactis* MG1363 was extracted as described previously. Plasmid DNA was isolated by the alkaline lysis procedure described by Sambrook *et al* (1989). Minipreparations of plasmid DNA from *E. coli* and *L. lactis* were made using the High Pure Plasmid isolation Kit from Roche, with minor modifications for *L. lactis*. Restriction enzymes and T4 DNA ligase were purchased from Roche. PCR amplifications were carried out using *Pwo* DNA polymerase for cloning fragments and *Taq* DNA polymerase for checking DNA insert sizes in plasmids from transformants. Electrotransformation of *E. coli* and *L. lactis* were performed with a Bio-Rad Gene Pulser (Bio-Rad Laboratories, Richmond, Calif.).

### Cloning of oppD promoter fragments

In order to study the regulation of promoter strength upstream of the *oppD* a deletion analysis of the respective promoters was carried out. Combinations of oligonucleotides opp 1 (5'
GCTCTAGACACTCACTTGTTTTGCTTCC 3')'
opp2 (5' AACTGCAGGAAAATTCATGAACATACC 3'),
opp1-opp3 (5' AACTGCAGTAAAACAATAATAAAAGCAG 3'),
opp1-opp4 (5' AACTGCAGGATAATAAAATTTGGACTG 3'),
opp1-opp14 (5' AACTGCAGCGTAATGTTCAGAAAATTC 3'),
opp1-opp15 (a) (5' AACTGCAGCGTAATATTTAGAAAATTCATGAACATACC 3') and
opp1-opp15 (b) (5' AACTGCAGCGTACTGTGCCGAAAATTCATGAACATACC 3') were used to amplify chromosomal DNA from *L. lactis* MG1363 in order to obtain fragments encompassing the *oppD* promoter. The PCR products were digested with *Xba*I and *Pst*I and transcriptionally fused upstream of the promoterless *lacZ* gene in the integration vector pORI13 (Sanders *et al*., 1998), also digested with the same enzymes. The resulting plasmids were called pORIopp2, pORIopp3, pORIopp4, pORIopp14, pORIopp15 (a) and pORIopp15 (b). All pORI constructions were preformed in *E. coli* EC101 which contains a chromosomal copy of the lactococcal *repA* gene needed for replication. They were then transformed into *L. lactis* LL108 and/or LL302 which contain multiple and single chromosomal copies of *repA*, respectively.

### Random mutagenesis of oppD promoter region

PCR fragments encompassing the *oppD* promoter region containing randomly introduced base pair substitutions were obtained essentially as described (Spee, de Vos, and Kuipers, 1993). Chromosomal DNA isolated from *L. lactis* MG1363 cultures were used as a template in the amplification step. Subsequently, the obtained variants were cloned into plasmid pORI13 and introduced into *L. lactis* LL108 as described above. Mutants showing distorted blue coloring on plates containing X-gal were selected and analyzed in more detail as described in results.

### Construction of a codY deletion strain.

A 1400 bp *Eco*RI/*Hin*DIII chromosomal fragment of *L. lactis* MG1363, containing *codY*, was subcloned in pUC19. The resulting plasmid was digested with *Snα*BI and subsequently selfligated. In this way, 423 bp were deleted from *codY*. The oligonucleotides cod280A (5' GGGAATTCGGATTGTCTATCTGCCTCG 3') and cod280B (5' GGGGGATCCAGATCTGACCATGATTACGCCAAGCTT 3') were used to amplify the Δ*codY*-containing fragment. PCR product was digested with *Eco*RI/*Bam*HI (restriction sites are underlined in the oligonucleotide sequence) and ligated into corresponding sites in pORI280. The resulting plasmid, pORIΔ*codY*, was introduced together with pVE6007 into *L. lactis* MG1363. As this strain does not contain the *repA* gene, selection for growth in the presence of erythromycin and increased temperature (37°C) forces pORIΔ*codY* to integrate into the chromosome by homologous recombination. A number of integrants were subsequently grown for about 30 generations under nonselective conditions allowing a second recombination event to occur, which results in either the deletion or the wild-type gene *codY.* The Δ*CodY* mutation was confirmed by PCR.

### β-Galactosidase activity assay

*In vivo* β-galactosidase (β-gal) assays were carried out in a Tecan microplate reader. Overnight cultures of *L*. *lactis* grown in GM17 were washed twice in 0.9% NaCl before inoculation to 2.5% in 200µl of the appropriate medium containing erythromycin (5µg/ml) for maintenance of pORI13 in *L. lactis* LL108/LL302 and erythromycin and chloramphenicol (2.5µg/ml each) (Leenhouts *et al*., 1996). The media also contained the β-gal substrate 2% β-trifluoromethylumbelliferyl β-D-galactopyranoside (Molecular probe T-657). Multilabelling experimental data (absorbance and fluorescence measurements) were processed using the Magellan software program. β-Gal production due to the transcription driven from the *oppD* upstream region was calculated as a function of light emission. β-Gal assays were performed throughout the growth of *L. lactis* grown in media in which growth rates differ significantly as a function of the nitrogen source i.e. CDM 0.2% casitone and CDM 2% casitone and measured of the culture.

### Overproduction and purification of His₆-CodY.

The chromosomaly located *codY* of *L. lactis* MG1363 was amplified by PCR with the oligonucleotides HC-5 (5' CTAGACCACCATGGGG *CATCACCATCACCATCAC*GTGGCTACATTACTTGAAAAAACACG 3'), introducing the underlined *Nco*I restriction enzyme site upstream of the hexa-histidine tag (italic) and
HC-6 (5' CTAGTCTAGAT*TAG*AAATTACGTCCAGCAAGTTTATC 3'),
introducing the underlined *Xba*I restriction enzyme site downstream of the stop codon (italic) of *codY*. The purified 833-bp PCR product was digested with *Nco*I and *Xba*I and ligated into the corresponding sites of pNZ8048, downstream of the nisin-inducible P_{nisA}. The resulting plasmid, pNH6CodY, was introduced in *L. lactis* NZ9000 to enable nisin induction of *his6-codY*, as described (de Ruyter, Kuipers, and de Vos, 1996;Kuipers *et al*., 1998). His6-CodY was isolated by affinity chromatography in an FPLC procedure (Amersham Pharmacia Biotech) using Ni-NTA agarose (Qiagen GmbH, Hilden, Germany).

### Electrophoretic mobility shift assays (EMSA's)

Gel retardation experiments were carried out essentially as described by Ebbole and Zalkin (Ebbole *et al*., 1989). Purified PCR products (2 µg) were end-labelled with polynucleotide kinase (Amersham Pharmacia Biotech) for 1 h at 37°C using 30 µCi [γ-³²P]-ATP (Amersham Pharmacia Biotech). Reactions were stopped by incubating the mixtures for 10 min at 70°C. Binding studies were carried out in 20 µl reaction volumes containing 20 mM Tris-HCl (pH 8.0), 8.7 % (v/v) glycerol, 1 mM EDTA (pH 8.0), 5 mM MgCl₂, 0.5 mM DTT, labelled DNA fragment (3000 cpm), and purified His6-CodY protein (50-400 ng). After incubation for 15 min at 30°C, samples were loaded onto a 4% polyacrylamide gel. Electrophoresis was performed in the Protean II Minigel System (Bio Rad) using a gradient (0.5x to 2x) of TAE buffer (Sambrook, Fritsch, and Maniatis, 1989) at 150 V for 1.5 h. Gels were dried and used for autoradiography at -80°C using Kodak XAR-5 films and intensifying screens.

### Preparation of cells for transcriptome analysis

Cells were grown at 30°C in GM17 supplemented with 0.5% glucose. Cells were grown till mid-exponential phase (OD600∼1.0). Approximately 5x10⁹ cells (50 ml culture) were harvested by centrifugation for 5 min at 10.000 rpm and 4°C. Cells were resuspended in 2 ml ice-cold growth media and divided over 4 screw-cap tubes with rubber seal. After the addition of 500 µl Phenol/Chloroform, 30 µl 10% SDS, 30 µl 3 M NaAc (pH5.2) and 500 mg glass-beads (diameter 75-150 µm), cells were frozen in liquid nitrogen and stored at -80°C or immediately used for RNA isolation.

### Transcriptome analysis

The DNA micro array experiments were essentially performed as described earlier (Kuipers *et al*., 2002), with the following modifications.

### RNA isolation

For each RNA isolation, one aliquot of the stored cell samples was used. Cells were disrupted by mechanical force using the Savant FastPrep FP120 system (Omnilabo) for 40 seconds at setting 5.0. Subsequently, RNA was extracted using the Roche "High Pure RNA Isolation Kit" according to the provided protocol. RNA yield and quality were determined spectrophotometrically and by performing a RNA 6000 Nano Labchip assay (Caliper Inc.) on the Agilent 2100 Bioanalyzer (Agilent Technologies, Amstelveen, the Netherlands) according to the manufacturers description respectively.

### cDNA labeling

Single-strand reverse transcription (amplification) and indirect labeling of 25 µg of isolated total RNA with either Cy3- or Cy5-dye were done with the Amersham CyScribe Post Labelling Kit according to the manufacturers protocol and, subsequently, used for hybridization.

### Hybridisation and scanning

Sylilated slides (Cel Associates) on which 2145 amplicons of *L. lactis* strain IL1403 were spotted in duplicate were used in the hybridization procedure. In addition, the slides contained 96 amplicons from *L. lactis* strain MG1363. Slides were pre-hybridized in Ambion SlideHyb buffer for 15 min at 40°C in a Genomic Solutions Hybstation. After removal of the pre-hybridization buffer, 10-25 µl of the Cy3/Cy5-labeled cDNA mix in 150 µl Ambion SlideHyb buffer I was added and incubated for 1 h at 42°C and finally for 16 h at 40°C. Afterwards, the hybridized slides were washed for 1 min in 2×SSC, 0.5% SDS and 5 min in 1×SSC, 0.25% SDS. The slides were scanned using a confocal laser scanner GeneTAC LS IV).

### Signal analysis

After scanning of the slides with the GeneTAC LS IV, individual spot intensities were determined. The raw data, along with the scanning image were stored in the Molecular Genetics Information System (MolGenIS). A grid definition was made to enable the spot analysis software Array Pro (Phoretix) to produce tables containing gene names and signal intensities. Using the program Excel (Microsoft corporation), signal intensities were corrected for background and the ratios in signal intensity between the different samples were determined.

### EXPERIMENTAL PART RESULTS

### CodY specifically binds to the oppD upstream region.

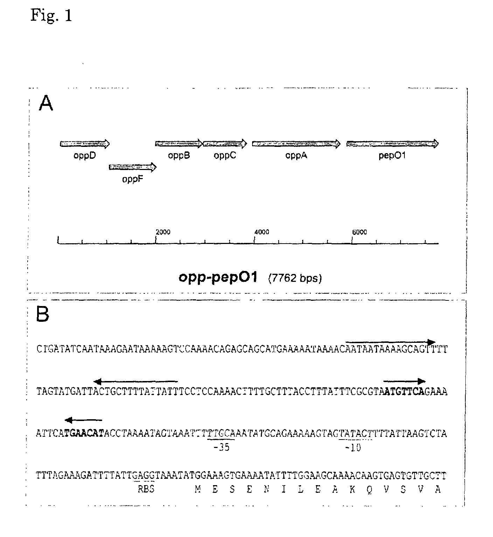

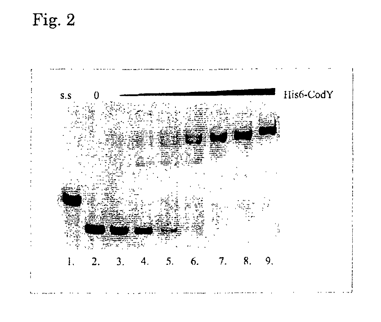

In order to examine whether *L. lactis* CodY directly interacts with upstream DNA sequences of its main target known so far, *oppD, in vitro* DNA binding studies were performed. For this purpose, histidine-tagged CodY was overexpressed using the nisin inducible gene expression system (Kuipers *et al*., 1998) and subsequently purified to apparent homogeneity. A radioactively labeled PCR fragment spanning the *oppD* upstream region (Fig. 1) was used as a probe. The electrophoretic mobility shift assays (EMSA's) clearly showed that purified H6-CodY is capable of binding directly to a region encompassing the *oppD* promoter (Fig. 2). Multiple retarded fragments were observed, indicating that, probably, multimerization of H6-CodY on the *oppD* promoter occurs.

### CodY regulates the expression of oppD by binding to a specific upstream sequence.

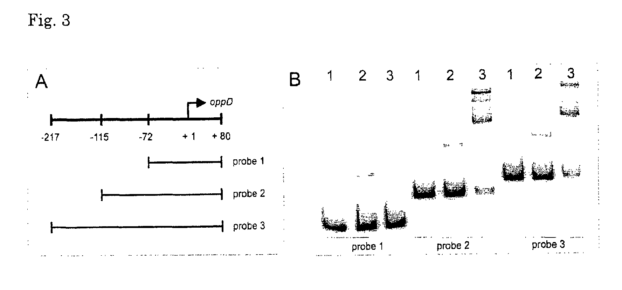

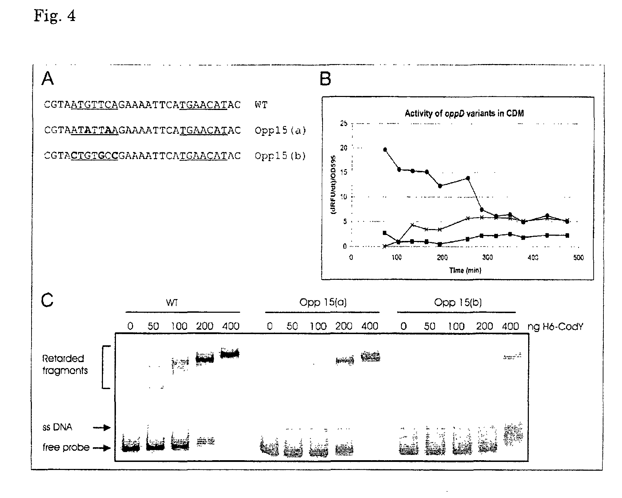

The upstream *oppD* region contains a small sequence that is inversely repeated with a spacing of 9 base pairs in between (Fig. 1). This region of dyad symmetry is located just upstream of the -35 promoter sequence. To determine whether this region is important for the interaction with CodY, a stepwise deletion analysis of the *oppD* upstream region was performed. Radioactively labeled deletion fragments of the region, obtained by PCR, were incubated with H6-CodY. As shown in Fig. 3, the fragments with an extended deletion of part of the *oppD* upstream region showed altered binding of CodY. As the fragments used in the EMSA's were also cloned upstream of the promoterless *lacZ* gene in plasmid pORI13, the *in vivo* regulation of a downstream reporter gene (*lacZ*) could be determined by performing β-galactosidase (β-gal) assays in a chemically defined medium (Poolman and Konings, 1988) containing 2% of casitone as a nitrogen source. It was shown that deletion of the sequence abolished medium dependent repression of *lacZ* expression (data not shown). The introduction of base substitutions in the upstream half-site of the inverted repeat gave rise to both weaker binding of H6-CodY and resulted in derepression of the promoter as shown by gel retardation analysis and β-gal assays, respectively (Fig. 4).

### Random mutation analysis of the oppD upstream region.

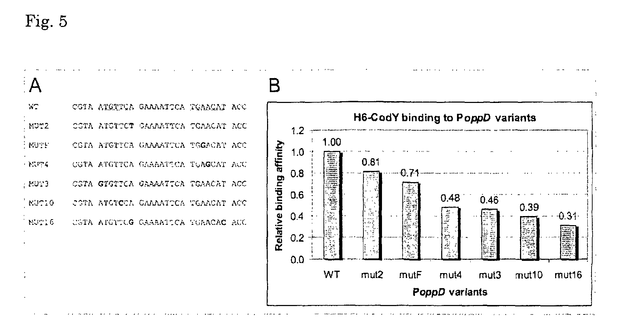

In order to identify bindings sites of CodY in the *oppD* upstream region, random mutation was carried out on the smallest *oppD* promoter fragment that is still bound by CodY (see Fig. 3). By choosing PCR conditions that allow mismatches to occur during DNA amplification, DNA fragments spanning the *oppD* promoter region were obtained containing randomly introduced base pair substitutions. The fragments were cloned upstream of the promoterless *lacZ* reporter gene in pORI13 and introduced in *L. lactis* LL108. Transformants that show a derepressed phenotype appear as white or light blue colonies on agarplates containing 2.5% of casitone and X-gal. Mutants that showed a distorted blue coloring on plates were tested for the ability to complex with CodY in a gel retardation experiment. The strength of binding was determined by comparing the amount of shifted mutated DNA fragment as compared to that of the corresponding wild type fragment (Fig. 5). Strikingly, all the mutants obtained carried substitutions in one or both of the half-sites of the inverted repeat present in the *oppD* upstream region, indicating the importance of this sequence in CodY binding.

### Identification of additional CodY targets.

In order to identify additional genes that are regulated by CodY, the transcriptional profile of wild-type (WT) *L. lactis* MG1363 was compared with that of a *L. lactis* MG1363 strain containing a 423 bps internal deletion in the *codY* gene using DNA micro arrays containing genes of *L. latis* IL1403. These studies revealed several differentially expressed genes in the delta *codY* strain. The genes of which the expression is increased most significantly in the delta *codY* strain are listed in Table 2. As this increased expression could be a direct effect of the absence of CodY (derepression), the upstream regions were examined for the presence of conserved nucleotide sequences. It was found that many of these genes contain an upstream sequence that is homologous to the upstream half-site of the palindromic sequence found to be important in *oppD* regulation (Fig. 6). Interestingly, the upstream region of the gene that shows the highest fold difference in expression when comparing the *codY* strain to WT *L. lactis* MG1363, *optS*, contains two copies of this sequence. The sequence identified is not in all instances part of an inverted repeat.

The conserved sequence could not be discerned in the upstream regions of all the differentially expressed genes. This could mean that the altered expression in the delta *codY* strain is an indirect effect of the mutation or that other sequences or structural determinants also play a role in the recognition/regulation by CodY. As can be seen in Fig.7 several other conserved motifs could be identified in the upstream regions of several differentially expressed genes. These sequences could play a role in CodY-regulated expression (e.g. in the case of *prtP* and *prtM*).

Using a weight matrix constructed from the sequences that comprise the putative CodY box, a search was performed in a data set containing the upstream regions of all IL1403 genes (Table 4). The presence of such an element could indicate that the downstream gene is under direct transcriptional control of CodY.

### Inter-species analysis of the over represented motif

A multi-species string search was performed on several Gram-positive bacteria in order to assess whether the putative CodY box found upstream of the genes belonging to the CodY regulon is also present in other bacteria containing a CodY protein. Such a comparison could reveal subtle differences in the consensus sequence of this motif. As depicted in Fig. 7, the comparison indeed showed that derivatives of the motif are present in the upstream sequences of CodY regulated genes of *B. subtilis* (e.g. *dppA* and *hutP*). Fig. 6 shows a graphical representation of a species-specific "weight matrix" that was built using the aligned sequences of *L. lactis* or *B. subtilis*, respectively. These matrices indicate the importance of specific bases at each position in the motif and show that the motif detected in *L. lactis* seems to be highly similar to the one of *B. subtilis.* Both encompass the same "core" sequence, but the consensus of the *L. lactis* motif seems to be somewhat extended on both sides.

Recently, Molle *et al*. (2003) reported the genome wide expression analysis of a *B. subtilis codY* mutant strain. Using the upstream nucleotide sequences of the targets found in that study, we searched for the presence of the putative CodY box in these sequences. Although the similarity scores with the consensus where not very high, derivates of the motif could be identified in some instances in this set of sequences (Table 5). Analysis of the full genome sequence of *B. subtilis* resulted in the identification of additional promoters containing the putative CodY box (Table 6).

A similar search was performed using the genomes of several other Gram-positive bacteria (Tables 7 and 8). Currently, we are mining the genome of *L. lactis* strain MG1363 of which the genome sequence is nearly completed.

### Regulation of prtP and prtM is derepressed in the CodY-deficient strain.

As *prtP* and *prtM* respond in a similar way to changes in the nitrogen content of the growth medium as CodY-regulated genes (Miladinov, Kuipers, and Topisirovic, 2001;Guedon *et al*., 2001a), the role of CodY in *prtP* and *prtM* expression was studied. A fragment containing the *prtP*/*prtM* intergenic region of *L. lactis* BGMN1-5 was cloned in between the promoterless *Escherichia coli* β-galactosidase (*lacZ*) and the *Cyamopsis tetragonoloba* α-galactosidase (*α-gal*) genes of pGKH10. Thus, translational fusions of *prtP* and *prtM* with the two reporter genes were created, the AUG codons of *prtP* or *prtM* serving as a start codon for *lacZ* or *α-gal*. In the resulting plasmid pGKB11, *lacZ* is under the control of the *prtP* promoter of BGMN1-5. In this plasmid the *prtM* promoter directs the transcription of the *α-gal* gene. In the corresponding plasmid pGKB12, the fragment is present in the opposite orientation. Plasmids pGKB11 and pGKB12 were introduced in the CodY-deficient *L. lactis* MG1363 strain (*L. lactis* MG1363*codY*Δ*1*) and *lacZ* expression was analysed in CDM media containing different amounts of casitone, and in peptide-rich M17 medium. In the WT strain, the β-gal activities of the *prtP*::*lacZ* and *prtM*::*lacZ* fusions were 6- and 8-fold lower in CDM with 2% than in CDM with 0.2% casitone, respectively. In the *codY* mutant, β-gal activity in CDM with 2% casitone was less than twofold lower than that in CDM with 0.2% casitone. β-gal activities in M17 were similar to those in CDM with 2% casitone for both MG1363 and MG1363*codY*Δ*1*. These results show that repression of these two gene fusions by medium peptides was almost fully abolished in the *codY* mutant.

### CodY binds to the prtP/prtM intergenic region.

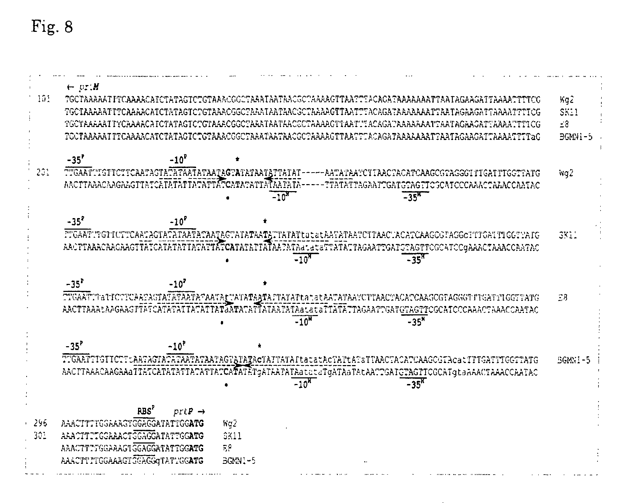

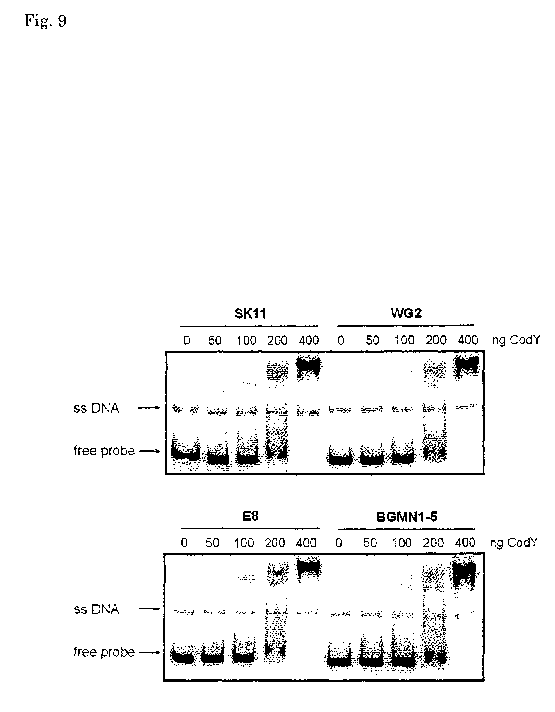

The *prtP*/*prtM* intergenic regions of the *L. lactis* strains BGMN1-5, E8, SK11 and Wg2 each contain an inverted repeat (Fig. 8), that is highly conserved on the sequential level (Marugg *et al*., 1996). To determine whether CodY is also able to recognize and bind to the *prtP*/*prtM* intergenic region, gel mobility shift assays were performed using purified H6-CodY. A 330-bp γ-³²P-labelled PCR fragment containing the *prtP*/*prtM* intergenic region derived from *L. lactis* BGMN1-5, E8, SK11 and Wg2, respectively, were used as probes. Addition of His6-CodY resulted in a markedly lower electrophoretic mobility of all four double stranded PCR products on a polyacrylamide gel as compared to the situation in which CodY was not added (Fig. 9). Moreover, multiple shifted bands are present suggesting that CodY migh act as a multimer. These results indicate that CodY directly binds to the *prtP*/*prtM* intergenic regions of all four lactococcal strains tested. Since the inverted repeats present in the *prtP*/*prtM* intergenic region overlaps the -10 sequence of both the *prtP* and *prtM* promoter, they could function as a binding site for CodY, thereby blocking transcription of both genes simultaneously.

### DESCIPTION OF FIGURES

**Figure 1** Overview of the *opp-pepO1* operon.
   Panel A shows a schematic overview of the *opp-pepO1 operon.* Panel B shows a detailed view of the upstream sequence encompassing the *oppD* promoter. The -35, -10 and ribosome binding site (RBS) sequences are underlined. The arrows show the positions of the two pairs of inverted repeats. The inverted repeat that is discussed in the text in more detail is indicated in bold.
**Figure 2** His6-CodY binds to the *oppD* promoter.
   Gel retardation assay using His6-CodY and the *oppD* promoter fragment. A DNA fragment encompassing the *oppD* promoter was amplified by PCR, radioactively labelled and incubated with no (lane 2) or increasing amounts of purified H6-CodY (lanes 3 to 9). Lane 1 contains the same probe, but was boiled in a 95% formamide solution in order to obtain single stranded DNA fragments.
**Figure 3** Determination of the minimal region involved in CodY binding to the *oppD* upstream promoter region.
   Different parts of the *oppD* promoter region were amplified by PCR (panel A), radioactively labelled and incubated with no (lanes 1), 20 ng (lanes 2) or 200 ng (lanes 3) of His6-CodY protein, respectively (panel B).
**Figure 4** Effects of site-directed mutations in the area of inverted repeat in the *oppD* upstream region. Three constructs (panel A) were compared for both β-gal activity (panel B) and H6-CodY binding (panel C). WT fragments contain no substitutions, whereas fragments Opp15 (a) and Opp15 (b) contain 2 and 3 mutations, respectively (indicated in bold in panel A). Solid lines mark the inverted repeats. Panel B shows the effects of mutations on *in vivo lacZ* expression during growth using WT (■), Opp15 (a) (×) and Opp15 (b) (•) *lacZ* fusion constructs. Activity is depicted as the change of fluorescence per unit time as function of the optical density.
**Figure 5** H6-CodY binding to fragments of derepressed variants of *lacZ* fusion constructs.
   Panel A shows the positions of the basepair substitutions (indicated in bold) of the mutants that showed distorted repression by CodY relative to the WT. Solid lines mark the inverted repeat. Panel B shows the relative binding of H6-CodY (compared to WT) to labelled PCR products encompassing the promoter region of the mutants in an *in vitro* binding assay. The relative affinity was calculated by comparing the amount of H6-CodY required to shift 50% of the labelled DNA in the binding assay.
**Figure 6** Weight matrices as deduced from the seperate motifs as observed in *L. lactis* intergenic (upper part) and *B. subtilis* (lower part). IUPAC codes of the derived consensus are indicated in bold.
**Figure 7** Several conserved motifs that can be discerned in the upstream regions of CodY-regulated genes. Motif 10 represents the consensus CodY target sequence as depicted in Figure 6.
**Figure 8** Comparison of the *prtP*/*prtM* promoter regions of *L. lactis* strains Wg2, SK11, E8 and BGMN1-5. The regions between bp 201 and 296 are shown double stranded for each strain, the other (flanking) regions are single stranded. Differences in the nucleotide sequences between the strains are depicted in lower case. *prtP* and *prtM* start codons are indicated in bold and with a small arrow. The deletion in the sequence of *L. lactis* Wg2 is indicated by a dashed line. The major transcription start sites for *prtP* (*) and *prtM* (•) are indicated above and below the sequence, respectively. Minor sites are indicated in bold face. Putative ribosomal binding sites (RBS^{P}, RBS^{M}) and -10 (-10^{P}, -10^{M}) and -35 (-35^{P}, -35^{M}) promoter sequences are overlined. Dashed arrows represent inverted repeats overlapping the promoters.
**Figure 9** Binding of H6-CodY to the *prtP*/*prtM* intergenic regions of *L. lactis* strains BGMN1-5, E8, SK11 and Wg2. The respective labelled DNA fragments were incubated with increasing amounts of CodY protein (as indicated above the gels) and subjected to gel electrophoresis. The positions of single stranded DNA (ss DNA) and free probe are indicated in the left margin.

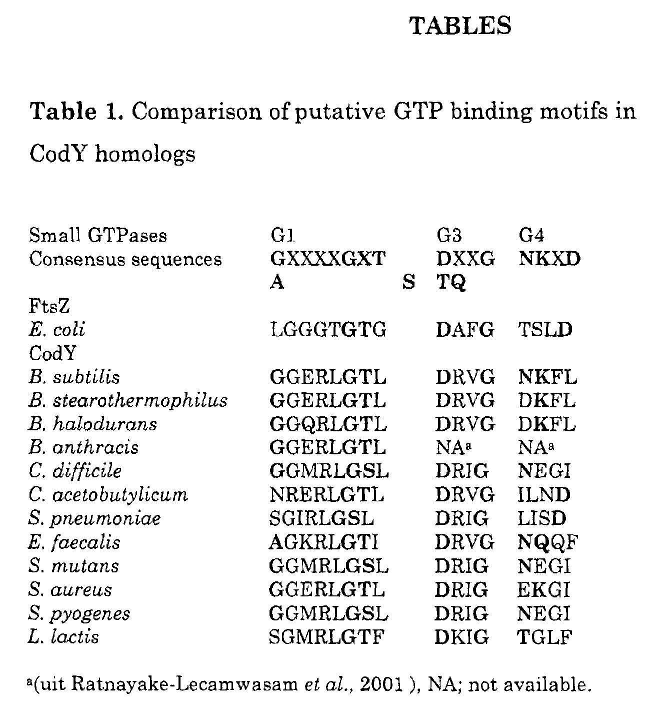

**Table 2.**

| Genes up in CodY strain | | |
|---|---|---|
| **Genes up in dcodY** | **Fold** | **Annotation** |
| optS | 8.8 | oligopeptide ABC transporter substrate binding protein |
| ctrA | 6.3 | cationic amino acid transporter |
| pepO | 5.7 | neutral endopeptidase |
| citB | 5.6 | aconitate hydratase (EC 4.2.1.3) |
| oppA | 5.3 | oligopeptide ABC trasporter substrate binding protein |
| gltD | 5.2 | glutamate synthase small subunit (EC 1.4.1.13) |
| ilvD | 4.8 | dihydroxy-acid dehydratase (EC 4.2.1.9) |
| ilvN | 4.5 | acetolactate synthase small subunit (EC 4.1.3.18) |
| asnB | 4.4 | asparagine synthetase B |
| ymdC | 3.9 | kanamycin kinase (EC 2.7.1.95) |
| hisA | 3.8 | phosphoribosylformimino-5-aminoimidazole isomerase |
| oppB | 3.4 | oligopeptide ABC trasporter permease protein |
| oppC | 2.9 | oligopeptide ABC trasporter permease protein |
| oppF | 2.8 | oligopeptide ABC trasporter ATP binding protein |
| llrH | 2.7 | two-component system regulator |
| hisB | 2.7 | imidazoleglycerol-phosphate dehydratase (EC 4.2.1.19) |
| serC | 2.6 | phosphoserine aminotransferase (EC 2.6.1.52) |
| hisK | 2.5 | histidinol phosphatase |
| oppD | 2.5 | oligopeptide ABC trasporter ATP binding protein |
| yahD | 2.4 | HYPOTHETICAL PROTEIN |
| leuC | 2.3 | 3-isopropylmalate dehydratase large subunit (EC 4.2.1.33) |
| optD | 2.3 | oligopeptide ABC trasporter ATP binding protein |
| serB | 2.2 | phosphoserine phosphatase (EC 3.1.3.3) |
| hisH | 2.1 | amidotransferase (EC 2.4.2.-) |
| udp | 2.1 | uridine phosphorylase |
| aldB | 2.0 | alpha-acetolactate decarboxylase (EC 4.1.1.5) |
| hisI | 2.0 | phosphoribosyl-ATP pyrophosphohydrolase (EC 3.6.1.31) |
| icd | 1.9 | isocitrate dehydrogenase (EC 1.1.1.42) |
| yafC | 1.9 | HYPOTHETICAL PROTEIN |
| arcD1 | 1.8 | arginine/ornitine antiporter |
| ilvB | 1.8 | acetolactate synthase large subunit (EC 4.1.3.18) |
| optF | 1.8 | oligopeptide ABC trasporter ATP binding protein |
| lacR | 1.8 | lactose transport regulator |
| hisD | 1.7 | histidinol dehydrogenase (EC 1.1.1.23) |
| arcC2 | 1.7 | carbamate kinase (EC 2.7.2.2) |
| optA | 1.7 | oligopeptide ABC transporter substrate binding protein |
| serA | 1.7 | D-3-phosphoglycerate dehydrogenase (EC 1.1.1.95) |
| recD | 1.7 | exodeoxyribonuclease V alpha chain (EC 3.1.11.5) |
| ilvC | 1.7 | ketol-acid reductoisomerase (EC 1.1.1.86) |
| ywaD | 1.7 | UNKNOWN PROTEIN |
| ydbD | 1.7 | UNKNOWN PROTEIN |
| glgD | 1.7 | glucose-1-phosphate adenylyltransferase (EC 2.7,7.27) |
| ImrP | 1.6 | integral membrane protein LmrP |
| pdhC | 1.6 | component of PDH complex (EC 2.3.1.12) |
| mesJ | 1.6 | cell cycle protein MesJ |
| lcnD | 1.6 | lactococcin A ABC transporter permease protein |
| glgA | 1.6 | glycogen synthase (EC 2.4.1.21) |
| trxA | 1.6 | thioredoxin |
| gltA | 1.6 | citrate synthase (EC 4.1.3.7) |
| pepN | 1.6 | aminopeptidase N |
| arcA | 1.6 | arginine deiminase (EC 3.5.3.6) |
| glgC | 1.6 | glucose-1-phosphate adenylyltransferase (EC 2.7.7.27) |
| yohC | 1.6 | transcriptional regulator |
| ywiE | 1.6 | UNKNOWN PROTEIN |
| yohD | 1.6 | UNKNOWN PROTEIN |
| yfiD | 1.5 | UNKNOWN PROTEIN |
| ycaF | 1.5 | UNKNOWN PROTEIN |
| ybhE | 1.5 | HYPOTHETICAL PROTEIN |
| rmlA | 1.5 | glucose-1-phosphate thymidylyltransferase (EC 2.7.7.24) |
| grpE | 1.5 | stress responce protein GrpE |
| yccE | 1.5 | UNKNOWN PROTEIN |
| hrcA | 1.5 | heat-inducible transcription repressor HrcA |
| pi336 | 1.5 | prophage pi3 protein 36 |
| dhaM | 1.5 | dihydroxyacetone kinase (EC 2.7.1.2) |
| ywjC | 1.5 | UNKNOWN PROTEIN |
| purC | 1.5 | phosphoribosylaminoimidazole synthetase |
| xynB | 1.5 | beta-1,4-xylosidase (EC 3.2.1.37) |
| lysA | 1.5 | diaminopimelate decarboxylase (EC 4.1.1.20) |
| yccF | 1.5 | HYPOTHETICAL PROTEIN |
| rmaB | 1.4 | transcriptional regulator |
| optC | 1.4 | oligopeptide ABC trasporter permease protein |
| araT | 1.4 | aromatic amino acid specific aminotransferase |
| yciA | 1.4 | amino acid amidohydrolase |
| pi235 | 1.4 | prophage pi2 protein 35 |
| yfgC | 1.4 | UNKNOWN PROTEIN |
| pepC | 1.4 | aminopeptidase C |
| yaiB | 1.4 | HYPOTHETICAL PROTEIN |
| ybhD | 1.4 | UNKNOWN PROTEIN |
| ribA | 1.4 | GTP cyclohydrolase II (EC 3.5.4.25) |
| pyrG | 1.4 | CTP synthetase |
| ps 120 | 1.4 | prophage ps1 protein 20 |
| yidA | 1.4 | transcription regulator |
| yahC | 1.4 | UNKNOWN PROTEIN |
| ps305 | 1.4 | prophage ps3 protein 05 |
| yndG | 1.4 | metal ABC transporter substrate binding protein |
| yahG | 1.4 | ABC transporter ATP binding protein |
| uxuA | 1.4 | D-mannonate dehydratase (EC 4.2.1.8) |
| ps112 | 1.4 | prophage ps1 protein 12 |
| hisC | 1.4 | histidinol-phosphate aminotransferase (EC 2.6.1.9) |
| arcC1 | 1.4 | carbamate kinase (EC 2.7.2.2) |
| xylX | 1.4 | acetyltransferase HYPOTHETICAL PROTEIN |
| rodA | 1.4 | rod-shape determining protein |
| yafB | 1.4 | sulfate transporter |
| ps113 | 1.4 | prophage ps1 protein 13 |
| ruvA | 1.4 | DNA helicase RuvA |
| asd | 1.4 | aspartate-semialdehyde dehydrogenase (EC 1.2.1.11) |
| sugE | 1.4 | SugE protein |
| groES | 1.4 | 10 KD chaperonin |
| rpsO | 1.4 | 30S ribosomal protein S15 |
| ybdA | 1.4 | transcription regulator |
| bcaT | 1.4 | branched-chain amino acid aminotransferase (EC 2.6.1.42) |
| ybiK | 1.4 | UNKNOWN PROTEIN |
| yafJ | 1.4 | HYPOTHETICAL PROTEIN |
| pi302 | 1.4 | prophage pi3 protein 02 |
| aldR | 1.4 | regulatory protein AldR |
| menX | 1.4 | protein in menaquinone biosynthesis pathway |
| dapB | 1.4 | dihydrodipicolinate reductase (EC 1.3.1.26) |
| yphI | 1.4 | UNKNOWN PROTEIN |
| ydcG | 1.3 | transcriptional regulator |
| amtB | 1.3 | ammonium transporter |

**Table 3.**

| Genes down in CodY strain | | |
|---|---|---|
| **Genes down in dcodY** | **Fold** | **Annotation** |
| plpA | 3.3 | outer membrane lipoprotein precursor |
| cysK | 3.2 | cysteine synthase (EC 4.2.99.8) |
| metB2 | 2.9 | cystathionine gamma-synthase (EC 4.2.99.9) |
| plpB | 2.9 | outer membrane lipoprotein precursor |
| cysD | 2.6 | O-acetylhomoserine sulfhydrylase |
| plpC | 2.5 | outer membrane lipoprotein precursor |
| plpD | 2.3 | outer membrane lipoprotein precursor |
| yndE | 2.3 | UNKNOWN PROTEIN |
| cysM | 2.1 | cysteine synthase (EC 4.2.99.8) |
| yrbB | 2.0 | HYPOTHETICAL PROTEIN |
| yjgC | 2.0 | amino acid ABC transporter substrate binding protein |
| argH | 1.8 | argininosuccinate lyase (EC 4.3.2.1) |
| lysP | 1.8 | lysine specific permease |
| gntK | 1.8 | gluconate kinase (EC 2.7.1.12) |
| codY | 1.7 | transcriptional regulator |
| yshA | 1.7 | amino acid permease |
| ymgI | 1.6 | UNKNOWN PROTEIN |
| ytaA | 1.6 | conserved hypothetical protein |
| panE | 1.6 | ketopantoate reductase (EC 1.1.1.169) |
| yhcE | 1.6 | conserved hypothetical protein |
| ywdD | 1.5 | UNKNOWN PROTEIN |
| ymbC | 1.5 | UNKNOWN PROTEIN |
| rpmD | 1.5 | 50S ribosomal protein L30 |
| ywjA | 1.5 | UNKNOWN PROTEIN |
| ynfG | 1.5 | HYPOTHETICAL PROTEIN |
| ybiD | 1.5 | HYPOTHETICAL PROTEIN |
| ydcB | 1.5 | amino acid ABC transporter ATP binding protein |
| ypjC | 1.5 | UNKNOWN PROTEIN |
| ytjE | 1.5 | aminotransferase |
| pydA | 1.5 | dihydroorotate dehydrogenase A (EC 1.3.3.1) |
| ymgG | 1.5 | HYPOTHETICAL PROTEIN |
| nrdG | 1.4 | ribonucleoside-triphosphate reductase activating protein |
| yxeA | 1.4 | HYPOTHETICAL PROTEIN |
| yueD | 1.4 | conserved hypothetical protein |
| ymbK | 1.4 | UNKNOWN PROTEIN |
| ylfH | 1.4 | N-acetylglucosamine catabolic protein |
| yqcA | 1.4 | UNKNOWN PROTEIN |
| yudI | 1.4 | HYPOTHETICAL PROTEIN |
| yvdF | 1.4 | amino acid ABC transporter substrate binding protein |
| yxaF | 1.4 | HYPOTHETICAL PROTEIN |
| ymbJ | 1.4 | UNKNOWN PROTEIN |
| ynfH | 1.4 | UNKNOWN PROTEIN |
| yxbE | 1.4 | conserved hypothetical protein |
| dxsA | 1.4 | 1-deoxyxylulose-5-phosphate synthase |
| murI | 1.4 | glutamate racemase (EC 5.1.1.3) |
| ytbC | 1.4 | UNKNOWN PROTEIN |
| yccB | 1.4 | UNKNOWN PROTEIN |
| yhcH | 1.4 | HYPOTHETICAL PROTEIN |

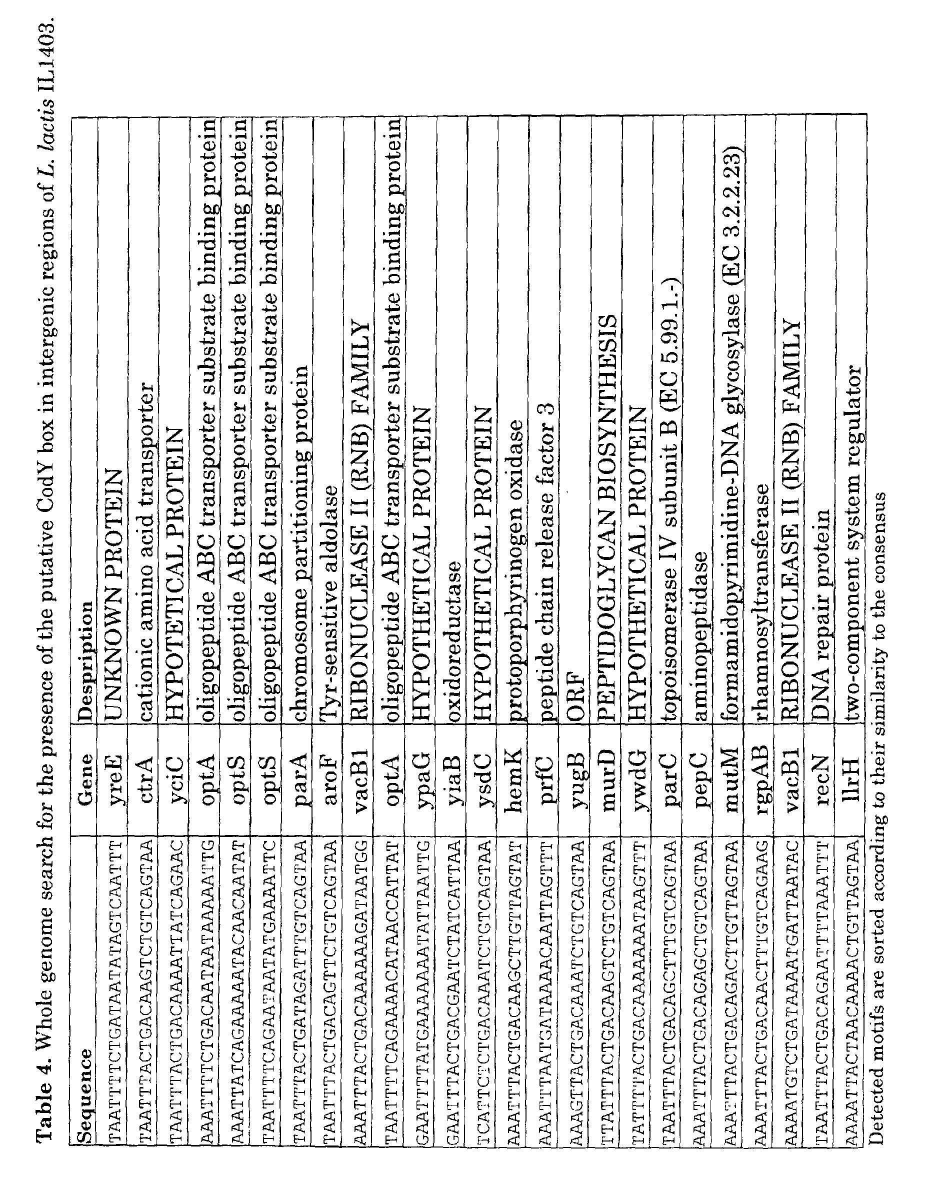

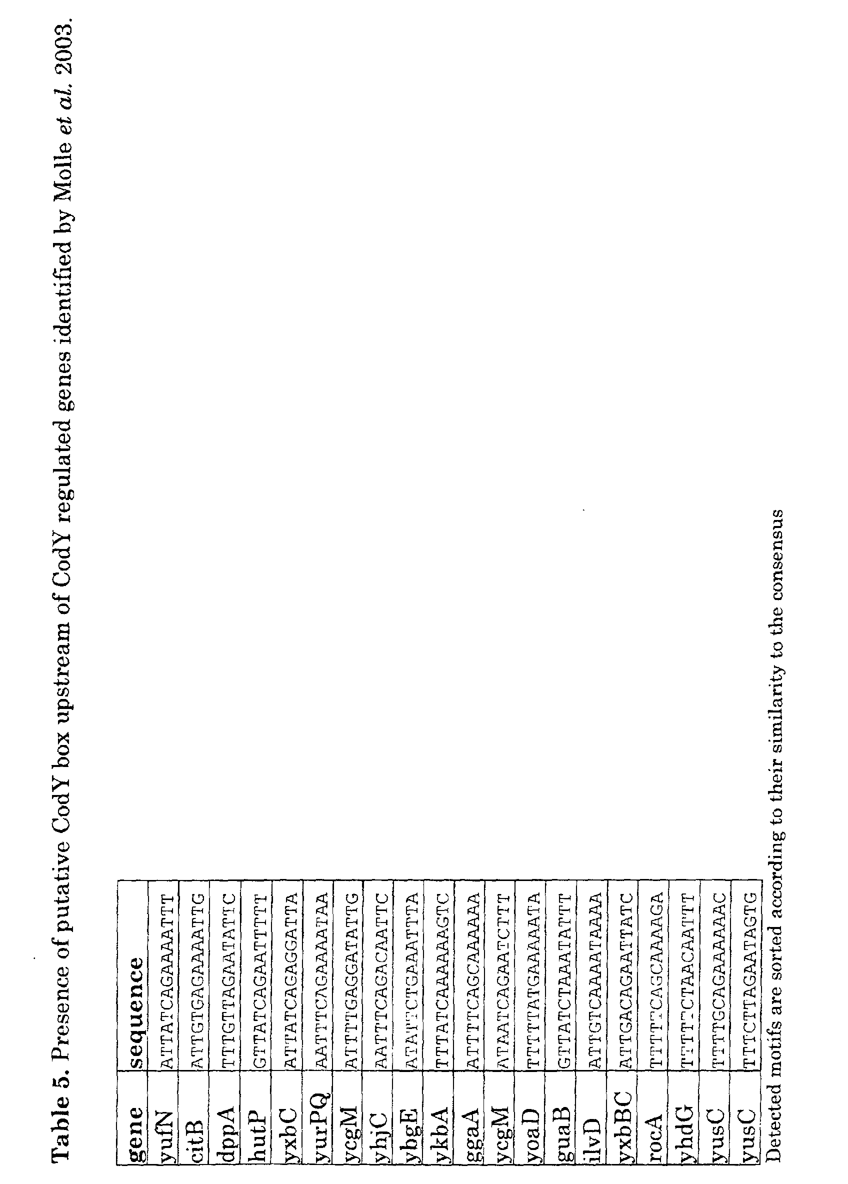

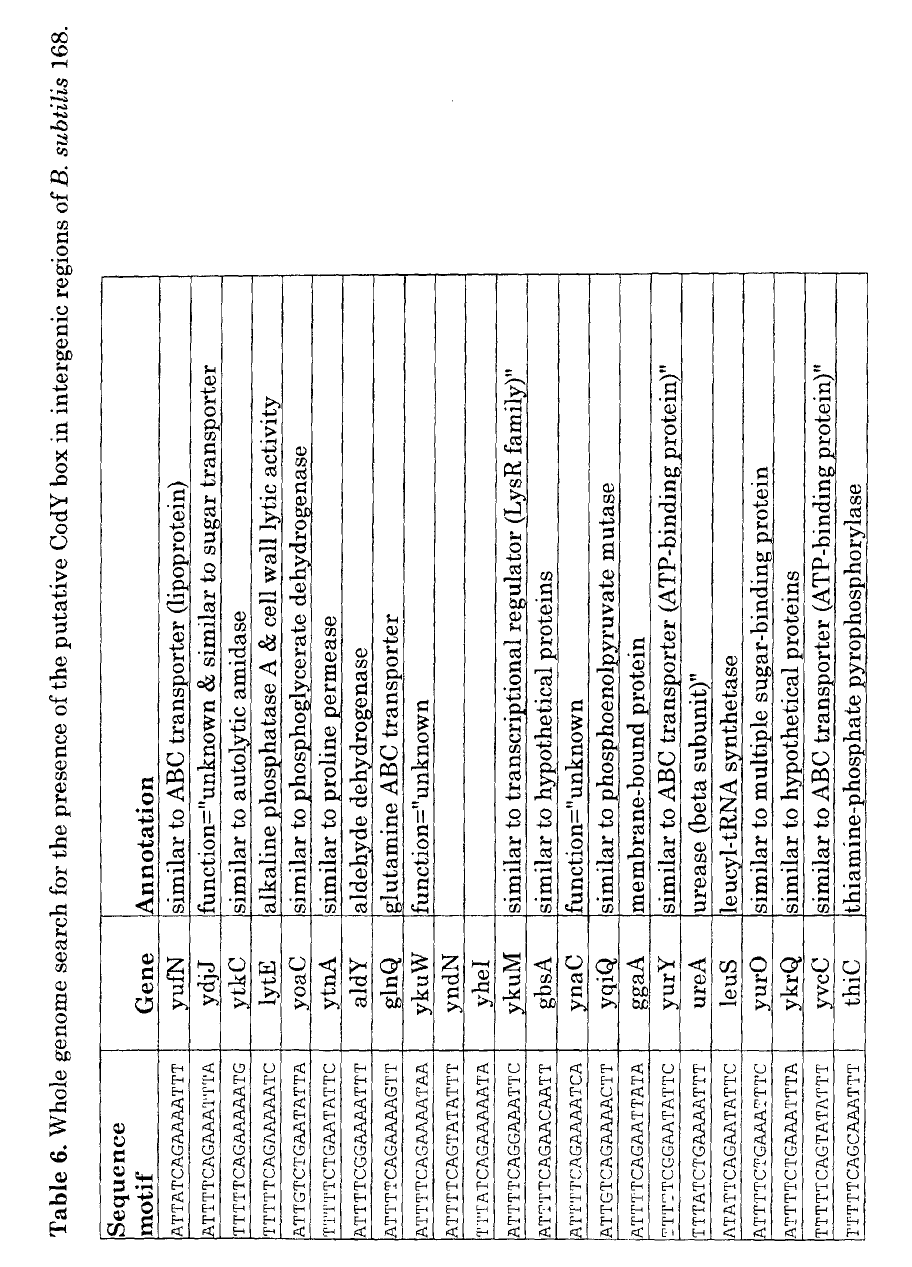

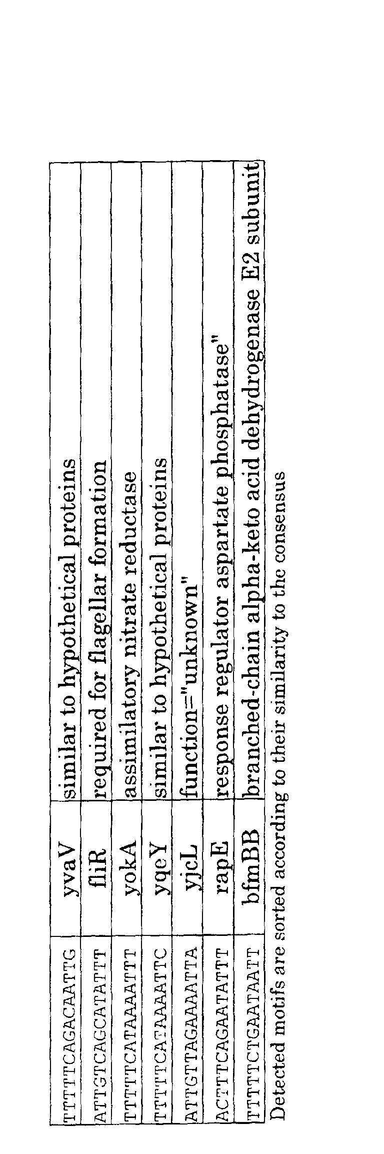

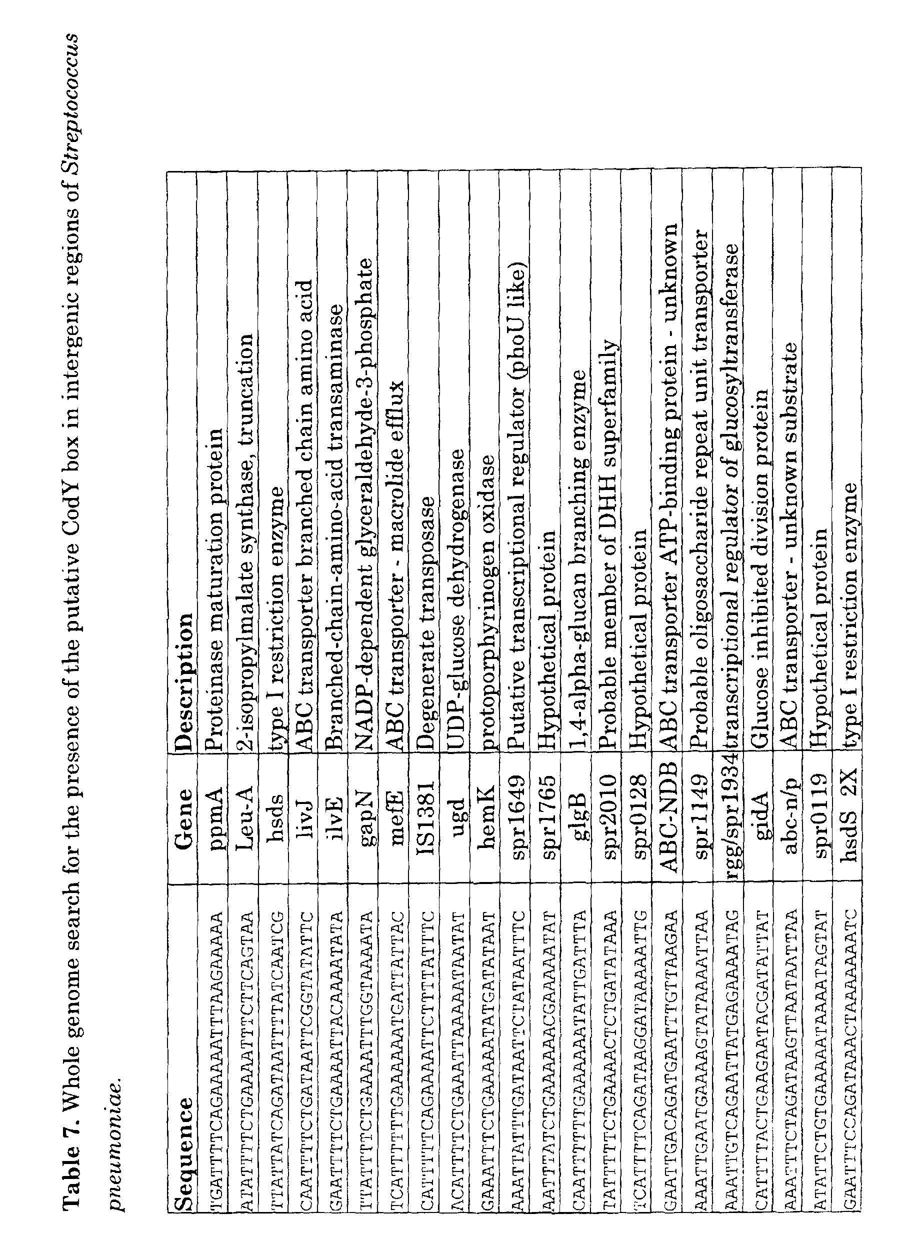

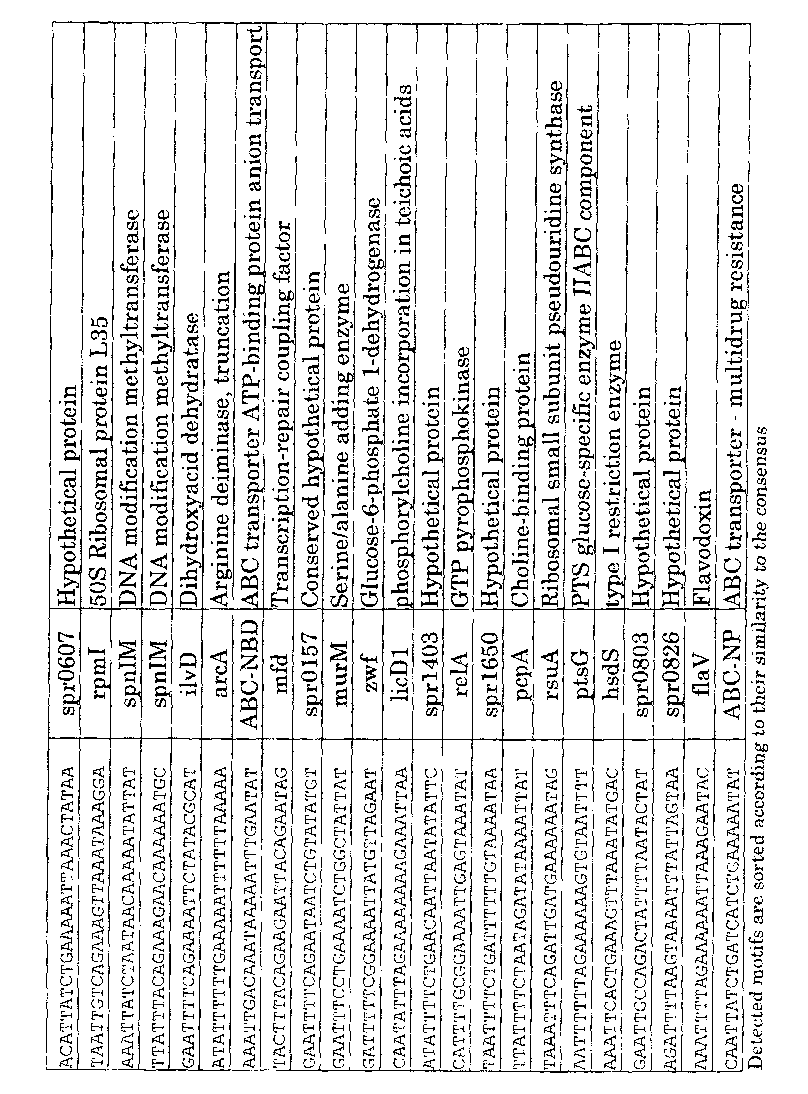

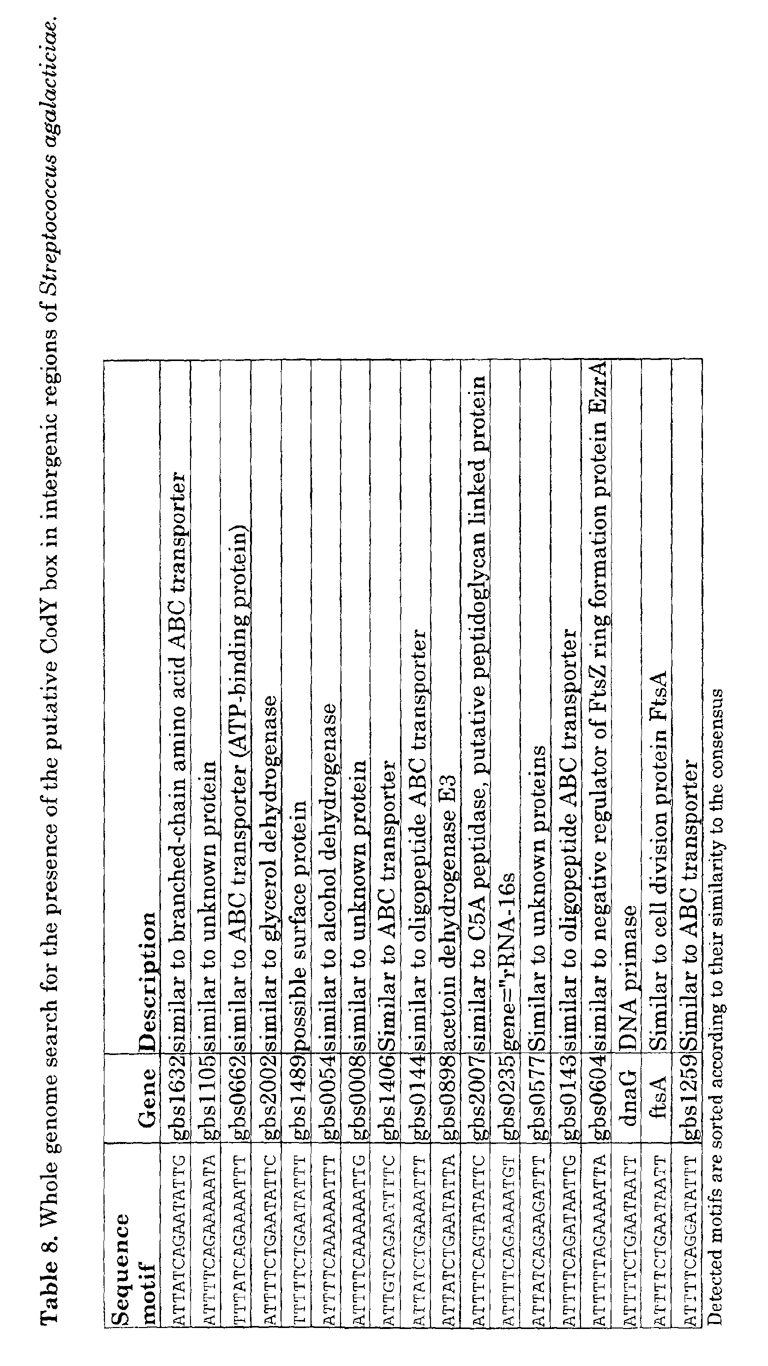

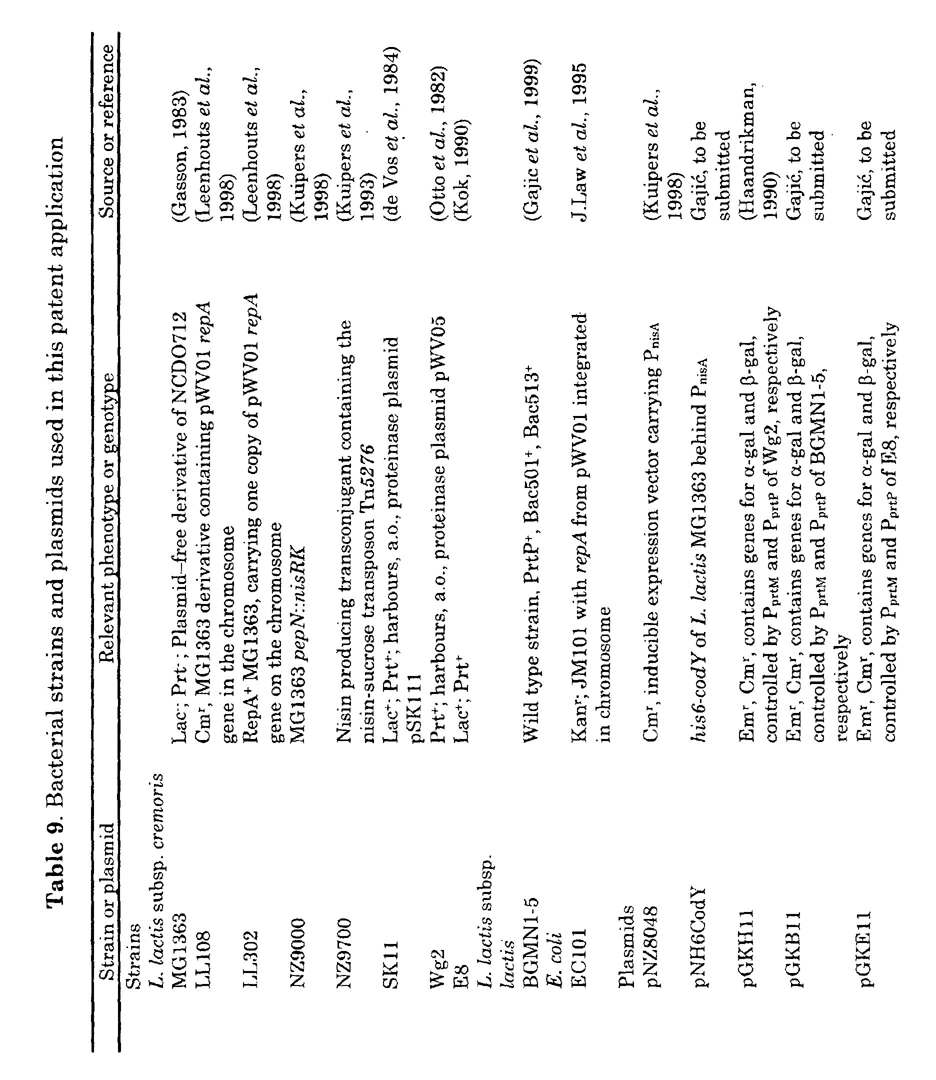

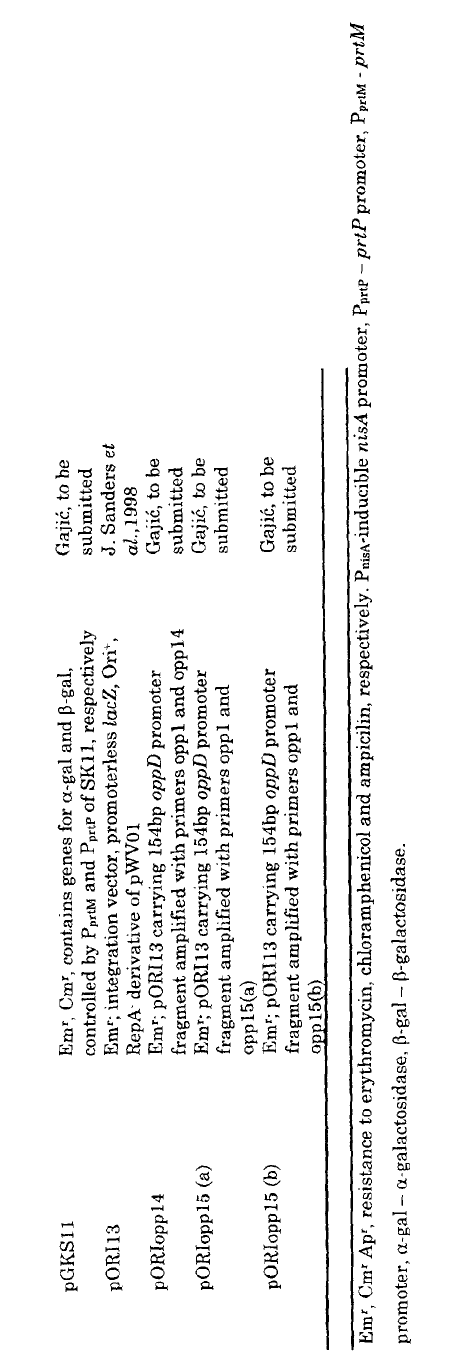

### REFERENCES

Alvarez,D., Novac,O., Callejo,M., Ruiz,M.T., Price,G.B., and Zannis-Hadjopoulos,M. (2002) 14-3-3sigma is a cruciform DNA binding protein and associates in vivo with origins of DNA replication *J.Cell Biochem.* 87: 194-207.

Christensen, J. E., Dudley, E. G., Pederson, J. A. and Steele, J. L. (1999). Peptidases and amino acid catabolism in lactic acid bacteria. *Antonie Van Leeuwenhoek* 76, 217-246.

Detmers, F. J., Kunji, E. R., Lanfermcijer, F. C., Poolman, B. and Konings, W. N. (1998). Kinetics and specificity of peptide uptake by the oligopeptide transport system of *Lactococcus lactis. Biochemistry* 37, 16671-16679.

Ebbole, D. J. and Zalkin, H. (1989). Interaction of a putative repressor protein with an extended control region of the *Bacillus subtilis pur* operon. *J.Biol.Chem.* 264, 3553-3561.

Exterkate, F. A. (1985). A dual directed control of cell wall proteinase production in *S. cremoris* AM1: a possible mechanism of regulation during growth in milk. *Journal of Dairy Science* 68, 562-571.

Fisher,S.H., Rohrer,K., and Ferson,A.E. (1996) Role of CodY in regulation of the Bacillus subtilis hut operon *J.Bacteriol*. 178: 3779-3784.

Foucaud, C., Kunji, E. R., Hagting, A., Richard, J., Konings, W. N., Desmazeaud, M. and Poolman, B. (1995). Specificity of peptide transport systems in *Lactococcus lactis*: evidence for a third system which transports hydrophobic di- and tripeptides. *J*.*Bacteriol*. 177, 4652-4657.

Gajic, O., Kojic, M., Banina, A. and Topisirovic, L. (1999). Characterization of natural isolate *Lactococcus lactis* subsp. *lactis* BGMN1-5, a strain producing two bacteriocins, cell wall-associated proteinase and showing clumping phenotype. *Arch.Biol.Sci.Belgrade* 51, 69-78.

Gasson, M. J. (1983). Plasmid complements of *Streptococcus lactis* NCDO 712 and other lactic streptococci after protoplast-induced curing. *J.Bacteriol.* 154, 1-9.

Guedon,E., Renault,P., Ehrlich,S.D., and Delorme,C. (2001a) Transcriptional pattern of genes coding for the proteolytic system of Lactococcus lactis and evidence for coordinated regulation of key enzymes by peptide supply *J.Bacteriol*. 183: 3614-3622.

Guedon,E., Serror,P., Ehrlich,S.D., Renault,P., and Delorme,C. (2001b) Pleiotropic transcriptional repressor CodY senses the intracellular pool of branched-chain amino acids in Lactococcus lactis *Mol.Microbiol*. 40: 1227-1239.

Haandrikman, A. J. (1990). Maturation of the cell envelope-associated proteinase of *Lactococcus lactis*. 64-71.

Kiwaki, M., Ikemura, H., Shimizu-Kadota, M. and Hirashima, A. (1989b). Molecular characterization of a cell wall-associated proteinase gene from *Streptococcus lactis* NCDO763. *Mol.Microbiol*. 3, 359-369.

Kok, J., van Dijl, J. M., van der Vossen, J. M. and Venema, G. (1985b). Cloning and expression of a *Streptococcus cremoris* proteinase in *Bacillus subtilis* and *Streptococcus lactis. Appl.Environ.Microbiol*. 50, 94-101.

Kok, J., Leenhouts, K. J., Haandrikman, A. J., Ledeboer, A. M. and Venema, G. (1988a). Nucleotide sequence of the cell wall proteinase gene of *Streptococcus cremoris* Wg2. *Appl*.*Environ*.*Microbiol*. 54, 231-238.

Kok, J. (1990). Genetics of the proteolytic system of lactic acid bacteria. *FEMS Microbiol.Rev.* 7, 15-42.

Kok, J. and de Vos,W.M. (1993) The proteolytic system of lactic acid bacteria. London: Blackie and professional, pp. 169-210.

Kuipers, O. P., Beerthuyzen, M. M., Siezen, R. J. and de Vos, W. M. (1993b). Characterization of the nisin gene cluster nisABTCIPR of Lactococcus lactis. Requirement of expression of the nisA and nisI genes for development of immunity. *Eur.J.Biochem*. 216, 281-291.

Kuipers,O.P., de Ruyter,P.G., Kleerebezem,M., and Vos,W.M. (1998) Qurum sensing controlled gene expression in lactic acid bacteria *Biotechnol* 64: 15-21.

Kunji, E. R., Mierau, I., Hagting, A., Poolman, B. and Konings, W. N. (1996). The proteolytic systems of lactic acid bacteria. *Antonie Van Leeuwenhoek* 70, 187-221.

Laan, H., Bolhuis, H., Poolman, B., Abee, T. and Konings, W. N. (1993). Regulation of proteinase synthesis in *Lactococcus lactis. Acta Biotechnology* 13, 95-101.

Leenhouts, K (1995) Integration strategies and vectors. *Dev. Biol. Stand.* 85: 523-530.

Leenhouts,K., Buist,G., Bolhuis,A., ten Berge,A., Kiel,J., Mierau,I., Dabrowska,M., Venema,G., and Kok,J. (1996) A general system for generating unlabelled gene replacements in bacterial chromosomes *Mol*.*Gen*.*Genet*. 253: 217-224.

Leenhouts, K., Bolhuis, A., Venema, G. and Kok, J. (1998b). Construction of a food-grade multiple-copy integration system for *Lactococcus lactis*. *Appl*.*Microbiol.Biotechnol*. 49, 417-423.

Marugg, J. D., Meijer, W., van Kranenburg, R., Laverman, P., Bruinenberg, P. G. and de Vos, W. M. (1995). Medium-dependent regulation of proteinase gene expression in Lactococcus lactis: control of transcription initiation by specific dipeptides. *J*.*Bacteriol*. 177, 2982-2989.

Marugg,J.D., van Kranenburg,R., Laverman,P., Rutten,G.A., and de Vos,W.M. (1996) Identical transcriptional control of the divergently transcribed prtP and prtM genes that are required for proteinase production in lactococcus lactis SK11 *J.Bacteriol*. 178: 1525-1531.

Meijer, W. C., Marrug, J. D. and Hugenholtz, J. (1996). Regulation of proteolytic enzyme activity in *Lactococcus lactis. Appl.Environ.Microbiol*. 62, 156-161.

Mierau, I., Haandrikman, A. J., Velterop, O., Tan, P. S., Leenhouts, K. L., Konings, W. N., Venema, G. and Kok, J. (1994). Tripeptidase gene (*pepT*) of *Lactococcus lactis*: molecular cloning and nucleotide sequencing of *pepT* and construction of a chromosomal deletion mutant. *J*.*Bacteriol*. 176, 2854-2861.

Miladinov,N., Kuipers,O.P., and Topisirovic,L. (2001) Casitone-mediated expression of the prtP and prtM genes in Lactococcus lactis subsp. lactis BGIS29 *Arch.Microbiol.* 177: 54-61.

Otto, R., de Vos, W. M. and Gavrieli, J. (1982). Plasmid DNA in *Streptococcus cremoris* Wg2: influence of pH on selection in chemostats of a varient lacking a protease plasmid. *Appl.Envion.Microbiol*. 43, 1272-1277.

Poolman,B., Konings,W.N. (1988) Relation of growth of Streptococcus lactis and Streptococcus cremoris to amino acid transport *J.Bacteriol.* 170: 700-707.

Ratnayake-Lecamwasam,M., Serror,P., Wong,K.W., and Sonenshein,A.L. (2001) Bacillus subtilis CodY represses early-stationary-phase genes by sensing GTP levels *Genes Dev.* 15: 1093-1103.

de Ruyter,P.G., Kuipers,O.P., and de Vos,W.M. (1996) Controlled gene expression systems for Lactococcus lactis with the food-grade inducer nisin *Appl.Environ.Microbiol*. 62: 3662-3667.

Sambrook,J., Fritsch,E.F., and Maniatis,T. (1989) *Molecular cloning: α laboratory manual* Cold Spring Harbor, N.Y.: Cold Spring Harbor laboratory Press.

Sanders,J.W., Venema,G., Kok,J., and Leenhouts,K. (1998) Identification of a sodium chloride-regulated promoter in Lactococcus lactis by single-copy chromosomal fusion with a reporter gene *Mol. Gen. Genet.* 257: 681-685.

Sanz,Y., Lanfermeijcr,F.C., Renault,P., Bolotin,A., Konings,W.N., and Poolman,B. (2001) Genetic and functional characterization of dpp genes encoding a dipeptide transport system in Lactococcus lactis *Arch.Microbiol*. 175: 334-343.

Serror,P., Sonenshein,A.L. (1996a) CodY is required for nutritional repression of Bacillus subtilis genetic competence *J*.*Bacteriol*. 178: 5910-5915.

Serror,P., Sonenshein,A.L. (1996b) Interaction of CodY, a novel Bacillus subtilis DNA-binding protein, with the dpp promoter region *Mol.Microbiol*. 20: 843-852.

Spee,J.H., de Vos,W.M., and Kuipers,O.P. (1993) Efficient random mutagenesis method with adjustable mutation frequency by use of PCR and dITP *Nucleic Acids Res*. 21: 777-778.

Tynkkynen,S., Buist,G., Kunji,E., Kok,J., Poolman,B., Venema,G., and Haandrikman,A. (1993) Genetic and biochemical characterization of the oligopeptide transport system of Lactococcus lactis *J.Bacteriol*. 175: 7523-7532.

Visser, S., Exterkate, F. A., Slangen, K. J. and De Veer, G. J. C. M. (1986). Comparative study of the action of cell wall proteinases from various strains of *Streptococcus cremoris* on bovine aₛ₁- b- and k-casein. *Appl.Environ.Microbiol*. 52, 1162-1166.

de Vos, W. M., Vos, P., de Haard, H. and Boerrigter, I. (1989). Cloning and expression of the *Lactococcus lactis* subsp. *cremoris* SK11 gene encoding an extracellular serine proteinase. *Gene* 85, 169-176.

## Claims

1. A method for regulating the expression of a gene of interest in a host cell that comprises a CodY-like protein comprising providing said cell with a gene of interest in operable linkage with a promoter and at least one CodY target sequence.

2. A method according to claim 1, wherein said promoter and/or said CodY target sequence is heterologous with regard to said gene of interest.

3. A method according to claim 1 or 2, wherein said CodY target sequence is heterologous with regard to said promoter.

4. A method according to any one of claims 1 to 3, wherein said gene of interest is a gene from a gram-positive bacterium.

5. A method according to any one of claims 1 to 4, wherein said gene of interest encodes a protease or a peptidase or an anti-microbial peptide or a vitamin.

6. A method according to any one of claims 1 to 5, wherein said CodY target sequence comprises a sequence as depicted in Figure 6 or a functional equivalent and/or a functional fragment thereof.

7. A method according to any one of claims 1 to 6, further comprising influencing the binding between said CodY-like protein and said at least one CodY target sequence.

8. A method according to claim 7, wherein said binding is regulated by subjecting said cell to a change in a growth condition.

9. A method according to claim 7 or 8, wherein said binding is regulated by subjecting said cell to a growth limiting condition.

10. A method according to claim 9, wherein said growth limiting condition is a limited availability of a nitrogen source.

11. A method according to any one of claims 1 to 10, wherein said host cell is a cell from a (diary) food production species.

12. A method according to claim 11, wherein said species is selected from a *Lactococcus* or *Lactobacillus* or *Streptococcus* or *Leuconostoc* or *Pediococcus* or *Bifidobacterium* or *Carnobacterium* or *Propionibacterium*.

13. A method according to any one of claims 1 to 12, wherein said host cell is provided with a nucleic acid encoding a CodY-like protein.

14. An isolated or recombinant nucleic acid that comprises at least one CodY target sequence or a functional fragment and/or a functional equivalent thereof.

15. A nucleic acid according to claim 14, further comprising a promoter in operable linkage with a gene of interest.

16. A nucleic acid according to claim 14 or 15 further comprising a gene encoding a CodY-like protein.

17. A nucleic acid according to claim 15 or 16, wherein said promoter and/or said at least one CodY target sequence is heterologous with regard to said gene of interest.

18. A nucleic acid according to any one of claims 15 to 17, wherein said CodY target sequence is heterologous with regard to said promoter.

19. A nucleic acid according to any one of claims 15 to 18, wherein said gene of interest is a gene from a gram-positive bacterium.

20. A nucleic acid according to any one of claims 15 to 19, wherein said gene of interest encodes a protease or a peptidase or an anti-microbial peptide or a vitamin.

21. A nucleic acid according to any one of claims 14 to 20, wherein said CodY target sequence comprises a sequence as depicted in Figure 6 or a functional equivalent and/or a functional fragment thereof.

22. A vector comprising a nucleic acid according to any one of claims 14 to 21.

23. A gene delivery vehicle comprising a nucleic acid according to any one of claims 14 to 21 or a vector according to claim 22.

24. A host cell comprising a nucleic acid according to any one of claims 14 to 21, a vector according to claim 22 or a gene delivery vehicle according to claim 23.

25. A host cell according to 22 which is a cell from a (dairy) food production species.

26. A host cell according to claim 24 or 25, wherein said species is selected from a *Lactococcus* or *Lactobacillus* or *Streptococcus* or *Leuconostoc* or *Pediococcus* or *Bifidobacterium* or *Carnobacterium* or *Propionibacterium*.

27. Use of at least one CodY target sequence for regulating the expression of a gene of interest.

28. A method for producing a (dairy) food product comprising a nucleic acid according to any one of claims 14 to 21, a vector according to claim 22, a gene delivery vehicle according to claim 23 or a host cell according to any one of claims 24 to 26.

29. A method according to claim 28, wherein said dairy product is a cheese or a fermented milk product

30. A cheese or a fermented milk product obtainable by a method according to claim 28 or 29.

31. A method for at least in part preventing the formation of off-flavours during a process for the production of a (dairy) food product, comprising providing at least one CodY target sequence upstream of a gene which product is, directly or indirectly, involved in the formation of off-flavours.

32. Use of a nucleic acid according to any one of claims 14 to 21 or a vector according to claim 22 or a gene delivery vehicle according to claim 23 or a host cell according to any one of claims 24 to 26 for increasing the expression of a gene of interest in a stationary phase culture or equivalents of said culture.
